(19)

Europäisches
Patentamt
European
Patent Office
Office européen
des brevets

(11) **EP 3 629 738 B1**

(12) **EUROPEAN PATENT SPECIFICATION**

(45) Date of publication and mention
of the grant of the patent:
**21.07.2021 Bulletin 2021/29**

(51) Int Cl.:
*A01N 59/06* [(2006.01)]  *A01N 59/08* [(2006.01)]
*A61K 33/06* [(2006.01)]  *A61K 33/14* [(2006.01)]
*A61K 35/08* [(2015.01)]  *A01K 61/13* [(2017.01)]

(21) Application number: **18727269.5**

(22) Date of filing: **24.05.2018**

(86) International application number:
**PCT/EP2018/063656**

(87) International publication number:
**WO 2018/219777 (06.12.2018 Gazette 2018/49)**

(54) **METHOD AND SYSTEM FOR TREATING FISH IN FISH FARMS**

VERFAHREN UND SYSTEM ZUR BEHANDLUNG VON FISCHEN IN FISCHFARMEN

PROCÉDÉ ET SYSTÈME DE TRAITEMENT DE POISSONS DANS DES FERMES PISCICOLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.05.2017 NO 20170878**

(43) Date of publication of application:
**08.04.2020 Bulletin 2020/15**

(73) Proprietor: **AkvaFresh AS
7125 Vanvikan (NO)**

(72) Inventors:
• **KOSBERG, Per
N-7030 Trondheim (NO)**
• **POWELL, Mark, Darryn
N-5268 Haukeland (NO)**

(74) Representative: **Onsagers AS
P.O. Box 1813 Vika
0123 Oslo (NO)**

(56) References cited:
EP-A2- 3 141 111        WO-A1-2008/069261
US-A1- 2013 056 417

• **MARK D. POWELL ET AL: "Freshwater treatment
of amoebic gill disease and sea-lice in seawater
salmon production: Considerations of water
chemistry and fish welfare in Norway",
AQUACULTURE, vol. 448, 1 November 2015
(2015-11-01), pages 18-28, XP055298507,
Amsterdam, NL ISSN: 0044-8486, DOI:
10.1016/j.aquaculture.2015.05.027**
• **IAN R. BRICKNELL ET AL: "Effect of
environmental salinity on sea lice
Lepeophtheirus salmonis settlement success",
DISEASES OF AQUATIC ORGANISMS, vol. 71,
no. 3, 1 August 2006 (2006-08-01) , pages 201-212,
XP055401585, ISSN: 1616-1580, DOI:
10.3354/dao071201**
• **B. M. CONNORS ET AL: "Effects of varying
salinities on Lepeophtheirus salmonis survival
on juvenile pink and chum salmon", JOURNAL
OF FISH BIOLOGY., vol. 72, no. 7, 1 May 2008
(2008-05-01), pages 1825-1830, XP055399367, NL
ISSN: 0022-1112, DOI:
10.1111/j.1095-8649.2008.01839.x**
• **S O MITCHELL ET AL: "A review of infectious gill
disease in marine salmonid fish", JOURNAL OF
FISH DISEASES., vol. 34, no. 6, 15 March 2011
(2011-03-15) , pages 411-432, XP055473588, GB
ISSN: 0140-7775, DOI:
10.1111/j.1365-2761.2011.01251.x**

EP 3 629 738 B1

- J STONE ET AL: "An evaluation of freshwater bath treatments for the control of sea lice, Lepeophtheirus salmonis (Kroyer), infections in Atlantic salmon, Salmo salar L.", JOURNAL OF FISH DISEASES., vol. 25, no. 6, 1 June 2002 (2002-06-01), pages 371-373, XP55397954, GB ISSN: 0140-7775, DOI: 10.1046/j.1365-2761.2002.00370.x
- POWELL MARK D ET AL: "In vitro survival and the effect of water chemistry and oxidative chemical treatments on isolated gill amoebae from AGD-affected Atlantic salmon", AQUACULTURE, ELSEVIER, AMSTERDAM, NL, vol. 220, no. 1, 14 April 2003 (2003-04-14), pages 135-144, XP009506696, ISSN: 0044-8486
- Harriet Perry ET AL: "Calcium concentration in seawater and exoskeletal calcification in the blue crab, Callinectes sapidus", Aquaculture, vol. 198, no. 3-4, 1 July 2001 (2001-07-01), pages 197-208, XP55760245, Amsterdam, NL ISSN: 0044-8486, DOI: 10.1016/S0044-8486(00)00603-7
- YUEFEI SONG ET AL: "Performance of UF NF integrated membrane process for seawater softening", DESALINATION, ELSEVIER, AMSTERDAM, NL, vol. 276, no. 1, 11 March 2011 (2011-03-11), pages 109-116, XP028230859, ISSN: 0011-9164, DOI: 10.1016/J.DESAL.2011.03.064 [retrieved on 2011-03-25]

**Description**

**Field of technology**

The present invention relates to a method and system for treating fish in fish farms.

**Background**

Farmed fish is an increasingly important protein source for humans. According to FAO, the world production of farmed fish reached about 66 million tonnes in 2014. This accounted for about 40 % of the total human fish consumption in that year. One important species in this respect is Atlantic salmon. The world production of Atlantic salmon in 2015 was above 2 million tonnes, of which more than half was produced in floating net-cages along the Norwegian coast.

The economy in fish farming speaks for having the fish populations in the fish farms as dense as possible. However, dense fish populations increase the risk for outbursts of infectious deceases and detrimental growth of parasitic life forms in the fish population. There is a range of ectoparasites, parasites adhering to and feeding from the skin and other exterior parts of the fish tissue. For example, two parasitic organisms that cause significant losses to the fish farming industry of salmonids around the world are sea-lice and amoeba causing amoebic gill disease (AGD).

Sea-lice are a family of parasitic copepods of which there are several species naturally occurring in seawater. Sea-lice spreads by releasing eggs which may float up to tens of kilometres in the surface region of the seawater and gradually develops into larvae. The larvae actively seek a host fish it may adhere to and develop into grown sea-lice. The sea-lice is an ectoparasite which eats skin, mucus and blood of the host fish and causes problems with increased susceptibility for developing infectious diseases, reduced growth, haemorrhaging of eyes and fins. The dominant sea-lice specie causing losses to Norwegian fish farmers is *Lepeophtheirus salmonis.*

Amoebic gill disease (AGD) is a potentially fatal disease caused by the amoeba *Neoparamoeba perurans,* which adheres to the gills of the host fish and causing problems with build-up of mucus on the gills and hyper-plastic lesions which gradually develops into deterioration of the gill tissue and severely compromising of the oxygen transport across the gill. Treatment costs for AGD-outbreaks are reported representing 10 - 15 % of the value of the fish stock for fish farms in Tasmania, and Australia.

**Prior art**

A common and widely applied method of reducing ectoparasites on fishes in fish farms is adding one or more antiparasitically active substances to the water containing the farmed fish to kill or paralyse the ectoparasites to make them release themselves from the host. This method has environmental issues such as spreading of antiparasitically active substances into the surroundings of the fish farms, fish health issues related to the fish being exposed to the antiparasitically active substances, and problems with reduced effect of the treatment over time due to the ectoparasites developing resistance towards the antiparasitically active substances.

Salmonids migrating from the ocean up in rivers to spawn lose many of their ectoparasites in a matter of hours up to a week or more while in the river water. Freshwater bathing has therefore been tried to treat salmonids for ectoparasites with good results. For example, is known from a report issued by the Norwegian Institute for Water Research [1] that bathing the salmonid fish for 3 to 4 hours in freshwater is effective in killing the amoebae *Neoparamoeba perurans.* This form of treatment has been applied in Tasmanian and Australian fish farms and shown to be effective in killing the AGD-causing amoeba.

Furthermore, research made by the Institute of Marine Research in Norway [2], has found that fresh water is particularly effective in almost immediate killing of young sea-lice at the copepodid stage (when the parasite is free swimming and actively seeking a host and to the moment when it attached to and lived a period on the host and moults into the first chalimus stage). However, after the sea-lice has moulted into the chalimus stages, it was observed that the lice managed to endure the fresh water for relatively long periods of up to eight days or more.

Protozoa living in water need to obtain, or at least be relatively close to, isotonic equilibrium with the surrounding water to cope with the osmotic pressure in their cytosol. For example, as discussed in Lima et al. 2015 [3], it is known that the cytosol of freshwater protists, such as flagellates, ciliates and amoeba, usually is hypertonic relative to their surroundings, such that the single-cell organisms must be able to handle permeation of water across their plasma membrane. This is obtained by a complex membrane-bound organelle know as contractile vacuole (CV) which prevents the single-celled organism from bursting by alternating cycles of diastoles and systoles to expel the excess water. The occurrence of CVs is reported common for marine ciliates; however, species of marine amoeba are commonly described as lacking such structures - and thus having limited ability to cope with reductions in salinity. Marine amoebas are thus assumed to exist in isotonic equilibrium with their environment.

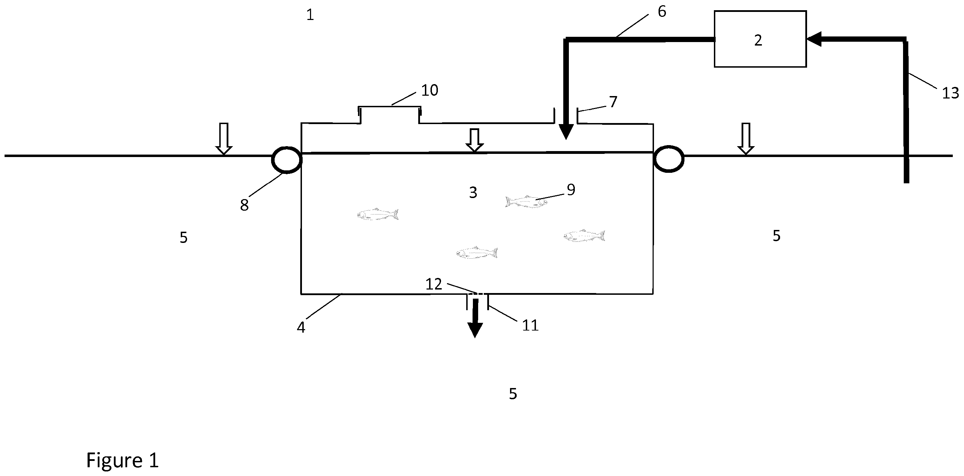

However, Lima et al. [3] has found that the AGD-causing amoeba *Neoparamoeba perurans,* has a similar osmoregulation mechanism as the freshwater protists which regulates its osmotic equilibrium towards the surrounding water by forming several contractile vacuoles in its cytoplasm. Lima et al. subject a sample of seawater having a colony of *Neoparamoeba perurans* to a sudden drop in salinity from 35 to 28 g/litre, i.e. a 20 % drop in salinity. It was observed that the amoeba immediately retracted and showed signs of stress. In about 5 minutes the amoeba displayed pseudopodia radiation and formed a visible contractile vacuole and contributory vacuoles. The main contractile vacuole is observed travelling towards the posterior of the cell and contributory vacuoles are observed fusing with the main vacuole and dispensing their content into the main vacuole. When the main vacuole reaches the cell membrane, it fuses with the cell membrane, expels the fluid inside the vacuole from the cell, and then the vacuolar membrane collapses. Afterwards the amoeba showed no vacuoles and regained normal shape and activity. It seems to have regained isotonic equilibrium with the diluted seawater. The process was observed to take about half an hour and is shown by a series of microphotographs presented in figure 1 of [3]. However, the above-mentioned observations that freshwater treatment effectively kills *Neoparamoebaperurans* show that their osmoregulation mechanism is not sufficiently efficient to cope with the osmotic pressure resulting from exposures to low salinities.

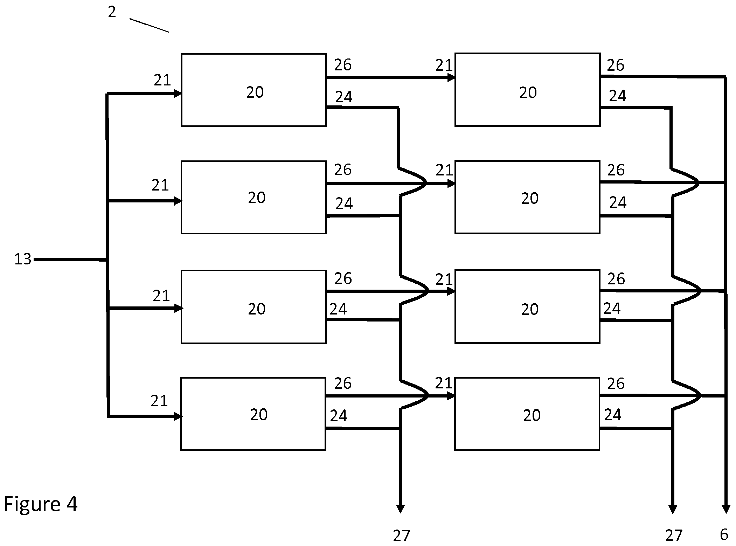

Green [6] discloses *in vitro* experiments made on the *Neoparamoebapemaquidensis* contained in artificially made seawater, natural seawater and natural seawater having added 10 g/L of $Na_2EDTA$. The artificial seawater was made (see e.g. table 4.2) by adding 35 g/L NaCl to fresh water and adding different amounts of $MgCl_2$ and $CaCl_2$ to make samples of artificial seawater having a range of dissolved Mg and Ca levels. These artificially made seawaters were applied to test the growth rate of *Neoparamoeba pemaquidensis* cultured in the water samples. Comparison experiments included the same growth tests in natural seawater and in a sample of natural seawater having added 10 g/L $Na_2EDTA$ which effectively chelated all $Mg^{2+}$ and $Ca^{2+}$ in the seawater. The artificial seawater samples had water hardness corresponding to 0, 138, 693, and 2773 mg/L $Ca^{2+}$. The growth rates after 24 hours culturing in these samples are presented in figure 4.5. Here it is seen that the samples of artificial seawater having 0 and 138 mg/L $Ca^{2+}$ showed a negative growth rate. The same did the sample of natural seawater with 10 g/L $Na_2EDTA$.

Mark [7] discloses *in vitro* experiments made on *Neoparamobeapermaquidensis* in freshwater containing various levels of $Na^+$, $Ca^{2+}$, and $Mg^{2+}$ levels. The experiments showed that the freshwater tolerance of amoebae isolated from the gills of AGD-affected Atlantic salmon exposed to water containing high levels of $Ca^{2+}$ or $Mg^{2+}$ (200 mg/L) had high levels of survival up to 3 hours of exposure. Exposure to water containing elevated $Na^+$, choline chloride or water at different pH all had no significant survival of amoebae. This work suggests that the hardness of freshwater may be an important factor for the survival of marine amoebae on the gills of AGD-affected salmon.

Perry et al. [8] discloses a study on the shedding of shells and calcification rates of shells of the blue crab, *Callinectes sapidus,* in seawater at various salinity levels and calcium contents. The study finds that salinity levels of 5, 12 and 25 ‰ do not influence the calcination rate provided the calcium concentrations were within the normal ranges for their respective salinities.Osmotic pressure in a diluted liquid is a function of the total amount of dissolved (chemical) species in the liquid as determined by the following expression [4];

$$\pi = MRT \qquad (1)$$

where $\pi$ is the osmotic pressure in the liquid, M is the total molarity of the dissolved species, R is the molar gas constant given in unit litre/atm·K, and T is the liquid temperature in Kelvin. Expression (1) gives that even relatively small differences in the content of dissolved species of e.g. 0.10 M (at a temperature of 25 °C) over a semi-permeable membrane, gives an osmotic pressure difference over the membrane of 2.45 atm. Sufficient to lift a water column 24.5 metres.

Yufei et al. [9] discloses a membrane system applying integrated ultra and nanofiltration membranes for softening seawater further comprising a permeate tank for softened seawater.

WO 2008/069261 discloses a water for artificial breeding (6) to be used for seawater organisms and freshwater organisms, which contains sodium, calcium and potassium by adding them to water for breeding such that it has a specific gravity of 1.004 or more and not more than that of natural seawater, and that the abundance ratio of potassium to calcium is from 0.93 to that in natural seawater and the abundance ratio of sodium to calcium and potassium is from 55 to that in natural seawater; and an aquaculture system using this water for artificial breeding, which includes a breeding tank (1) placed in a space at an ambient temperature between 15°C and 30°C in the surface of the ground, the water for artificial breeding (6) filled in the breeding tank (1), and a filtering device (2) for filtering the water for artificial breeding (6) filled in the breeding tank (1).

From NO 334487 it is known an arrangement for forming a treatment zone inside a floating saltwater fish farm by placing a floating container being closed at the top but which has one or more openings in the bottom where fish at will may swim from below into the container, and at least one inlet for freshwater at the top for filling the interior of the container with freshwater. The container provides thus a confined volume/treatment chamber inside the floating saltwater fish farm where the fish may be exposed to freshwater.

From NO 333846 it is known system and arrangement for treating salmonids with freshwater in saltwater floating net-cage fish farms. The freshwater is produced by having one or more floaters in the surface zone which each is anchored to the seabed and equipped with a hydraulic pump which due to the surface wave action of the sea pressurises an amount of seawater. The pressurised seawater is then passed on to a reverse osmosis membrane to be desalinated and form freshwater. The freshwater is then passed into a treatment area/zone inside the floating net-cage. The document suggests two embodiments of the treatment area/zone inside the floating net-cage. The first is a floating circular structure having a channel which is filled with the freshwater. The other embodiment comprises forming the freshwater zone at the top layer of the floating net-cage by having a cage skirt/tarpaulin or other "wall" running along the upper peripheral part of the floating net-cage preventing the seawater from mixing with the water inside the "wall".

In commercial fish farming the number of fish to be treated, the size of the fish, and location of net pens pose may cause significant logistical limitations. One significant issue in this regard is the amount of fresh water required to give the entire fish population in large-scale commercial fish farming regular baths in freshwater and the distance to the nearest freshwater source. The energy requirement for desalination of seawater by e.g. reversed osmosis is in the order of 20 kJ per litre produced freshwater, leading to considerable energy costs for producing the required amounts of fresh water if the fish farm is located at places unsuited for supply of naturally formed freshwater.

From US 2012/0152721 it is known that divalent ions such as e.g. $Ca^{2+}$ and $Mg^{2+}$ may be selectively extracted from seawater by use of nanofiltration.

**Objective of the invention**

It is an objective of the present invention to provide a method and system in that the softened seawater having a salinity in the range from 1.0 to 15 g/kg and a $Ca^{2+}$ content of < 100 mg/ kg is used as medicament for reducing calcium sensitive ectoparasites on a marine fish, preferably one or more of: Neoparamoeba perurans or Lepeophtheirus salmonis.

It is further an objective to provide a method and system in that the softened seawater with a salinity in the range from 1.0 to 15 g/kg and a $Ca^{2+}$ content of < 100 mg/kg is used as an effective treatment of salmonids in fish farms for sea-lice and/or amoebic gill disease.

**Description of the invention**

The invention is based on a discovery made by the present inventor that the killing effect of water having salinity in the range from 1.0 to 15 g/kg on the amoeba *Neoparamoeba perurans* is related to the concentration of $Ca^{2+}$-ions in the water and not only the total content of dissolved salts. In experiments performed by the inventor, it is observed killing of the amoeba when exposing them to water having relatively high contents of dissolved salts comparable to seawater levels, but with a lowered concentration of $Ca^{2+}$-ions as compared to seawater. This discovery is surprising and contradicts prior knowledge of osmoregulation in protozoa because this knowledge indicates that it is the osmotic pressure resulting from the exposure to low salinities which kills the organisms.

Thus, in a first aspect, the invention relates to a softened seawater for use as a medicament, characterised in that the softened seawater has:

a salinity in the range from 1.0 to 15 g/kg, determined as the total mass of $Na^+$, $K^+$, $Ca^{2+}$, $Mg^{2+}$, $Cl^-$, $HCO_3^-$, $CO_3^{2-}$, and $SO_4^2$ ions being present in a sample of one kg of the softened seawater, and

- a $Ca^{2+}$ content of $\leq$ 100 mg/kg, determined as the mass of $Ca^{2+}$ ions in a sample of one kg of the softened seawater,

and in that

- the softened seawater is used as medicament for reducing calcium sensitive ectoparasites on a marine fish, preferably one or more of: *Neoparamoeba perurans* or *Lepeophtheirus salmonis.*

In a second aspect, the present invention relates to softened seawater having:
a salinity in the range from 1.0 to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and

- a $Ca^{2+}$ content of $\leq$ 100 mg/kg, determined as the mass of $Ca^{2+}$ ions in a sample of one kg of the softened seawater,

for use in a method for treating marine fish for calcium sensitive ectoparasites by containing the fish for a period of time in the softened seawater, wherein the fish to be treated is a salmonid, preferably Atlantic salmon (*Salmo salar*) and the method is aimed at reducing *Neoparamoeba perurans* or *Lepeophtheirus salmonis.*

In a third aspect, the present invention relates to a system for treating marine fish for calcium sensitive ectoparasites, wherein the system comprises:

- a supply unit (2) of softened seawater having;

    - a salinity in the range from 1.0 g/kg to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
    - a $Ca^{2+}$ content of $\leq$ 100 mg/kg, determined as the mass of $Ca^{2+}$ ions in a sample of one kg of the softened seawater,

    and

- a treatment zone (3) containing a volume of the softened seawater dimensioned to contain and sustain an intended number of marine fishes to be treated for a period of time,

and wherein the treatment zone is one of:

- a container at least partly filled with the softened seawater located either in-situ in a marine fish farm (14, 16) or external to the marine fish farm (14, 16),
- a top layer of a floating net-cage (14, 16) formed by a cage skirt/tarpaulin running along the upper peripheral part of the floating net-cage and where the top layer of water in the net-cage is softened seawater, or
- a barge or boat floating outside the net cages of marine fish farms having at least one compartment/container filled with softened seawater.

The $Ca^{2+}$-content of the softened seawater applied in the invention according to the first to fourth aspect of the invention may advantageously be in one of the following ranges; from 0.001 to 95 mg/kg, from 0.001 to 90 mg/kg, from 0.001 to 80 mg/kg, from 0.001 to 50 mg/kg, from 0.001 to 10 mg/kg, from 0.001 to 8 mg/kg, from 0.001 to 6 mg/kg, from 0.001 to 5 mg/kg, from 0.001 to 4 mg/kg, or most preferred from 0.001 to 2.5 mg/kg, determined as the mass of $Ca^{2+}$-ions in a sample of one kg of the softened seawater. That is, a $Ca^{2+}$-content of e.g. 0.1 mg/kg means that in a sample of water of one kg, the mass of the $Ca^{2+}$-ions being dissolved in that water sample is 0.1 mg.

The salinity of the softened seawater applied in the invention according to the first to the fourth aspect of the invention may advantageously be in one of the following ranges; from 1 g/kg to 15 g/kg, from 1 g/kg to 12.5 g/kg, from 1 g/kg to 10 g/kg, from 2 g/kg to 15 g/kg, from 2 g/kg to 12.5 g/kg, or from 2 g/kg to 10 g/kg, or most preferably from 2 g/kg to 8 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater.

The term "softened seawater" as used herein, is defined to be any water, regardless of its origin, comprising dissolved salts in an amount corresponding to a salinity in the range from 1.0 g/kg to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and dissolved calcium corresponding to a $Ca^{2+}$-content of $\leq$ 100 mg/kg, determined as the mass of $Ca^{2+}$-ions in a sample of one kg of the softened seawater. Thus, even though it is especially preferred to use natural seawater and adjusting its salt content to these levels as the softened seawater, this is not mandatory. The present invention may apply any water having a salt content within the above specified salinity and calcium ion content.

The term "salt" as used herein means any ionic compound resulting from a neutralisation reaction of an acid and a base. Naturally occurring water has a wide variety of dissolved salts at a very wide range of concentrations making it practically challenging to determine the amount of salt being dissolved in the water. However, the most common ions in naturally occurring water is $Na^+$, $K^+$, $Ca^{2+}$, $Mg^{2+}$, $Cl^-$, $HCO_3^-$, $CO_3^{2-}$, and $SO_4^{2-}$. Thus, the term "salinity" as used herein is defined to be the combined mass of $Na^+$, $K^+$, $Ca^{2+}$, $Mg^{2+}$, $Cl^-$, $HCO_3^-$, $CO_3^{2-}$, and $SO_4^{2-}$ ions being present in a unit sample of water. Thus, a salinity of e.g. 0.5 g/kg means that in a sample of 1 kg water, 0.5 gram is the combined mass of the $Na^+$, $K^+$, $Ca^{2+}$, $Mg^{2+}$, $Cl^-$, $HCO_3^-$, $CO_3^{2-}$, and $SO_4^{2-}$ ions being present in the sample.

Freshwater is usually defined as water having a salinity of less than 0.5 g/kg. Water having salinities above this level is often termed as brackish water. Thus, the term "freshwater" as used herein is defined to be water having a salinity of less than 0.5 g/kg. The term "brackish water" as used herein is defined to be water having a salinity in the range from 0.5 g/kg to 30 g/kg. Natural seawater has typically a salinity in the range from 30 g/kg to 40 g/kg. Seawater in the North Sea usually has a salinity of around 35 g/kg (and a calcium content of around 400 mg/kg).

The treatment according to aspect one to four of the invention may advantageously be as simple as making the fishes swimming in and/or dwelling for a period of time in a volume of softened seawater having the salinity and calcium content as specified in the first aspect of the invention. Thus, the term "containing the fish in water" as used herein, simply means that the fish is present in a volume of softened seawater having the salinity and calcium content as specified in the first aspect of the invention for a sufficient time to harm ectoparasite species being present on the fish and which is sensitive to the calcium content of the water such that it either releases itself from their host or dies.

The term "treatment zone" as used herein, means any volume of the softened seawater dimensioned to contain and sustain an intended number of fishes to be treated against calcium sensitive ectoparasites for a period of time sufficient to achieve the intended treatment. The volume of softened seawater of the treatment zone may be obtained by any known or conceivable method for obtaining water of the salinity and calcium content falling within the above definition of softened seawater, and the volume of softened seawater may be contained to form the treatment zone in any known or conceivable way, including but not limited to, filling a chamber, container, tank, or any other confinement able to hold water.

The system according to the third aspect of the invention may in one example embodiment, advantageously further comprise a transport mechanism for transporting fish to and from the treatment zone. The transport mechanism may be any known or conceivable solution known to the skilled person for transporting living fishes to be treated into the volume of softened seawater of the treatment zone and to extract living fishes out of the volume of softened seawater of the treatment zone without causing any significant bodily harm or health problem to the fish.

The term "calcium sensitive ectoparasites" as used herein, means any marine species that may live, wholly or part of its lifecycle, as a parasite on the skin/gills and other surface of a marine fish and which is sensitive to calcium depleted water according to the softened seawater of the invention.

The term "period of time" as used herein encompasses any practically obtainable bathing time of the fishes to be treated, ranging from very short periods of a few seconds up to practically continuous treatment. The treatment according to the invention, i.e. the period of time the fish needs to be contained in the water according to the invention may vary significantly depending on which marine fish species being treated, which ectoparasite specie(s) the treatment is aimed at removing, intended ratio of removal of the ectoparasites, water temperature, salinity and/or $Ca^{2+}$ content of the water being applied, and other factors. Thus, the period of bathing time in the water according to the invention may vary from a few seconds up to many days, but can easily be established by the person skilled in the art by trivial trial and error tests. In practice, the length of the period of time the fish is to be contained in the water according to the invention may advantageously be in one of the following ranges; from 5 minutes to 200 hours, preferably from 5 minutes to 100 hours, more preferably from 10 minutes to 48 hours, more preferably from 20 minutes to 36 hours, more preferably from 30 minutes to 30 hours, more preferably from 60 minutes to 24 hours, and most preferably from 4 hours to 24 hours.

The treatment according to aspect one to four of the invention may be applied on both wild marine fish and on farmed marine fish species. However, the practical implementation of the invention will often be in connection with marine fish farming, such that the treatment zone may advantageously be implemented *in-situ* or in proximity of a marine fish farm in any known or conceivable manner known to the skilled person. Alternatively, the treatment zone may be separate from and located at a distant location from a fish farm.

For net-cage based marine fish farms, which presently is the most common type of fish farm in commercial fish farming, the treatment zone necessarily needs to be formed separate from the breeding/living quarter of the farmed fishes, but may be located both inside or separate from the net-cage. Thus, example embodiments of suited treatment zones include, but is not limited to; a floating container located inside a net cage as shown in e.g. NO 334487 filled with the softened seawater, forming the treatment zone as the top layer of the floating net-cage by having a cage skirt/tarpaulin or other "wall" running along the upper peripheral part of the floating net-cage and filling the volume inside the "wall" with softened seawater, a barge or boat floating outside the net cages of marine fish farms having at least one compartment/container filled with softened seawater as a treatment zone, etc.

In a further alternative, the treatment zone may be the permanent living quarters for the farmed fishes. I.e. that the fishes are not contained in separate breeding tanks having natural seawater and then transported to and from a treatment zone having the softened seawater, but that the breeding tanks of the fish farm are filled with softened seawater such that the fishes are permanently treated against calcium sensitive ectoparasites. This alternative is applicable for land-based fish farms and for fish farms carried on boats/barges etc., i.e. fish farms having fish tanks isolated from the sea.

The current conventional approach for fighting ectoparasites in the fish farming industry is treating the fish for ectoparasites after identifying clinical signs of ectoparasite related disease in the fish population. One advantage of the present invention is that it is suited for prophylactic treatment, i.e. treating the fish population on non-clinical schedules. This enables treating the fish population when the ectoparasite is at an infectious stage instead of waiting until the ectoparasites reach their mature reproductive stage as in the current conventional approach. A prophylactic approach has the advantage of a significantly increased suppression of the ectoparasite growth and population size in the fish farm environment as compared to the conventional approach which awaits the eruption of a disease before treating the fish. A prophylactic treatment may typically involve regularly or intermittently repeated treatments of at least part of the fishes in each treatment living on a fish farm to keep the growth of the ectoparasite colony on the fishes in check while promoting optimal fish health and animal welfare. The intervals at which a fish is subject to the prophylactic treatment according to the invention may advantageously be at regular intervals of from once a week to once a year, at regular intervals from once every two weeks to once every six months, at regular intervals once every month to once every fourth month, or at regular intervals once every second month to once every third month.

A particularly preferred method for producing the softened seawater for use in aspect one to four of the present invention is membrane based nanofiltration of seawater. A nanofiltration membrane is a porous membrane which selectively retains molecules and solid particles in a liquid from passing through the membrane due to a sieving effect (mechanical retention) determined by the pores size. A nanofiltration membrane typically has pore sizes in the range from 1 to 10 nm. Due to the pore sizes of nanofiltration membranes a relatively large fraction of divalent ions, such as e.g. $Ca^{2+}$-ions, in the seawater is prevented from passing through the membrane, i.e. being retained in the retentate. In comparison, a relatively larger fraction of the physically smaller monovalent ions such as e.g. $Na^+$, $K^+$ etc. can pass through the nanofiltration membrane. The permeate flow consists therefore of seawater having strongly reduced contents of divalent ions and only moderately reduced contents of monovalent ions, such that nanofiltration membranes are well suited for making the softened seawater for use in the invention according aspect one to four of the invention. Furthermore, a nanofiltration membrane may also utilise the Gibbs-Donnan effect (electrochemical retention) to increase the selectivity in retaining charged particles and ions by incorporating charged molecules in the membrane matrix. Anionic nanofiltration membranes (having fixed positive charges in the membrane matrix) will, in addition to the sieving effect towards the relatively larger divalent ions, also retain divalent anions such as $Ca^{2+}$ due to the electric repulsion forces between the anions and the fixed positive charges in the membrane matrix. This Gibbs-Donnan effect will be more effective towards divalent anions than monovalent anions, and thus increase the selectivity of the membrane towards the dissolved salt in seawater. Nanofiltration membrane modules with neutral or anionic membranes and which are suited for use in the present invention are commercially available.

The present invention according to aspect one to four may apply any nanofiltration membrane module able to retain a significant fraction of the $Ca^{2+}$-content of the seawater and produce a softened seawater as the permeate. The nanofiltration membrane module being applied should advantageously be able to produce softened seawater having the required low $Ca^{2+}$-contents in one passing, i.e. the seawater is filtered in only a single membrane module. However, this is not mandatory because membrane modules may be assembled in series such that the permeate flow of the first membrane module is passed on and injected as inlet flow of the second membrane module such that the seawater is subject to two subsequent filtrations. There may be applied any number of nanofiltration membrane modules assembled in series to obtain the intended low calcium contents of the permeate to qualify as softened seawater for use in the present invention. The amount of produced softened seawater may be tailored to satisfy any thinkable need associated with fish farming by assembling two or more nanofiltration membrane modules in parallel.

The invention according to any of aspect one to four may treat any marine fish species against any ectoparasite species which are sensitive towards lowered calcium levels. Preferably, the invention according aspect one to four may advantageously be applied to treat farmed salmonids against *Lepeophtheirus salmonis* and/or AGD-causing amoeba *Neoparamoeba perurans.*

An especially preferred example embodiment of the invention according to aspect one to four is treating salmonids, such as e.g. Atlantic salmon (*Salmo salar*) for AGD, i.e. the ectoparasite to be removed is *Neoparamoebaperurans.*

When the invention according to any of the first to the fourth aspect is applied for treating salmonids for AGD-causing amoeba, the softened seawater may preferably comprise dissolved salts in an amount of:

- a salinity in the range from 1.0 g/kg to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
- a $Ca^{2+}$ content of less than 50 mg/kg, determined as the mass of $Ca^{2+}$ ions in a sample of one kg of the softened seawater.

More preferably, the salinity of the softened water for treating salmonids for AGD-causing amoeba may be in one of the following ranges: from 1 g/kg to 12.5 g/kg, from 1 g/kg to 10 g/kg, from 2 g/kg to 10 g/kg, or from 2 g/kg to 8 g/kg, and the $Ca^{2+}$-content of the softened seawater for treating salmonids for AGD-causing amoeba may be in one of the following ranges: from 0.001 to 30 mg/kg, from 0.001 to 15 mg/kg, from 0.1 to 10 mg/kg, from 0.1 to 8 mg/kg, from 0.1 to 6 mg/kg, from 0.1 to 4 mg/kg, or most preferred from 0.1 to 2.5 mg/kg.

The salmonids exposure time to the softened seawater for treatment of AGD-causing amoeba may preferably be in the range from 1 hour to 48 hours, more preferably from 1 hour to 36 hours, more preferably from 2 hours to 24 hours and most preferably from 4 hours to 24 hours. An example embodiment of a pre-emptive scheme for keeping the AGD-causing amoeba population in check may advantageously be exposing the salmonids (i.e. containing the fish in the softened seawater) to the softened for one of the above exposure lengths once every sixth month, preferably every fourth month, more preferably every third month, more preferably every second month, more preferably every month, or most preferably every second week.

Another especially preferred example embodiment of the invention is treating salmonids, such as e.g. Atlantic salmon (*Salmo salar*) for sea-louse, i.e. the ectoparasites to be removed are *Lepeophtheirus salmonis* and/or various *Caligus* species. The treatment according to the invention is most effective when the sea-louse is in the copepod stages, such that the treatment of the invention may preferably be repeated at regular intervals to keep the growth and spreading of the sea-lice in the fish farm in check.

When the invention according to any of aspects one to four is applied for treating salmonids for sea-louse, such as e.g. *Lepeophtheirus salmonis* and/or various *Caligus* species, the softened seawater may preferably comprise dissolved salts in an amount of:

- a salinity in the range from 1.0 g/kg to 10 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
- a $Ca^{2+}$ content of $\leq$ 20 mg/kg, determined as the mass of $Ca^{2+}$ ions in a sample of one kg of the softened seawater.

More preferably, the salinity of the softened water for treating salmonids for sea-louse may be in one of the following ranges: from 1 g/kg to 9 g/kg, from 1.5 g/kg to 8 g/kg, from 1.5 g/kg to 7 g/kg, or from 2 g/kg to 7 g/kg, and the $Ca^{2+}$-content of the softened seawater for treating salmonids for seal-louse may be in one of the following ranges: from 0.1 to 20 mg/kg, from 0.1 to 15 mg/kg, from 0.2 to 10 mg/kg, from 0.2 to 6 mg/kg, from 0.2 to 5 mg/kg, from 0.2 to 4 mg/kg, or most preferred from 1 to 3 mg/kg.

The salmonids exposure time to the softened seawater for treatment of sea-louse may preferably be in the range from 1 hour to 48 hours, more preferably from 1 hour to 36 hours, more preferably from 2 hours to 24 hours and most preferably from 4 hours to 24 hours. An example embodiment of a pre-emptive scheme for keeping the sea-louse population in check may advantageously be exposing the salmonids (i.e. containing the fish in the softened seawater) to the softened for one of the above exposure lengths once every sixth month, preferably every fourth month, more preferably every third month, more preferably every second month, more preferably every month, or most preferably every second week.

The method according to the fourth aspect may advantageously apply a softened seawater having a $Ca^{2+}$ content is in one of the following ranges from 0.001 to 95 mg/kg, from 0.001 to 90 mg/kg, from 0.001 to 80 mg/kg, from 0.001 to 50 mg/kg, from 0.001 to 10 mg/kg, from 0.001 to 8 mg/kg, from 0.001 to 6 mg/kg, from 0.001 to 5 mg/kg, from 0.001 to 4 mg/kg, or most preferred from 0.001 to 2.5 mg/kg, determined as the mass of $Ca^{2+}$ ions in a sample of one kg of the softened seawater.

**List of figures**

Figure 1 is a schematic cut-view drawing as seen from the side of an example embodiment of the system according to the invention.

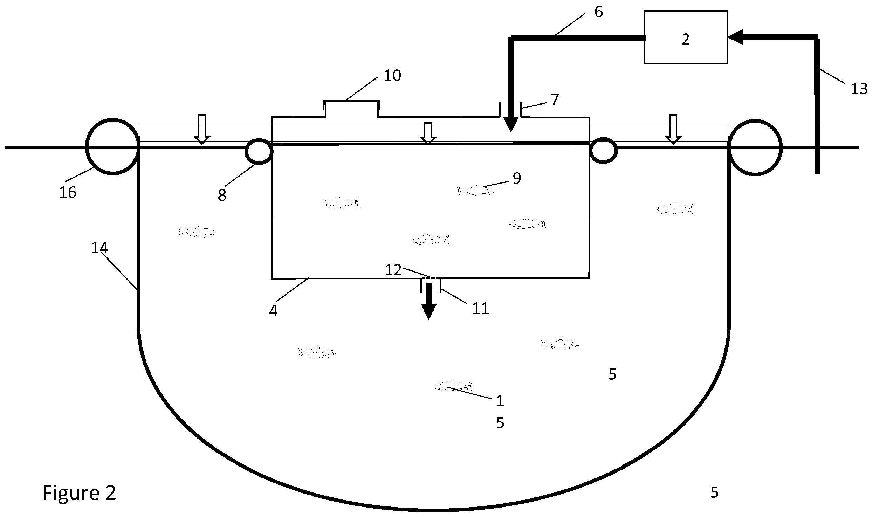

Figure 2 is a schematic cut-view drawing as seen from the side of an example embodiment of how the system according to the invention shown in figure 1 may be incorporated in a fish farm.

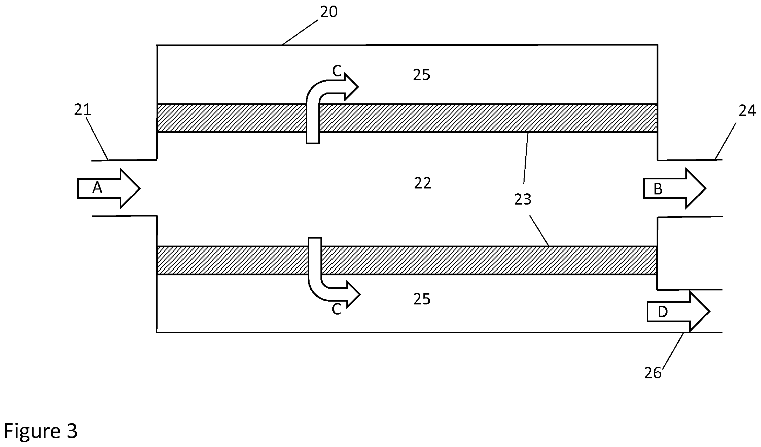

Figure 3 is a schematic cut-view drawing as seen from the side of an example embodiment of a nanofiltration membrane module for production of softened seawater.

Figure 4 is diagram illustrating an example embodiment of an assembly of nanofiltration membrane modules comprising four parallel rows of two membrane modules assembled in series.

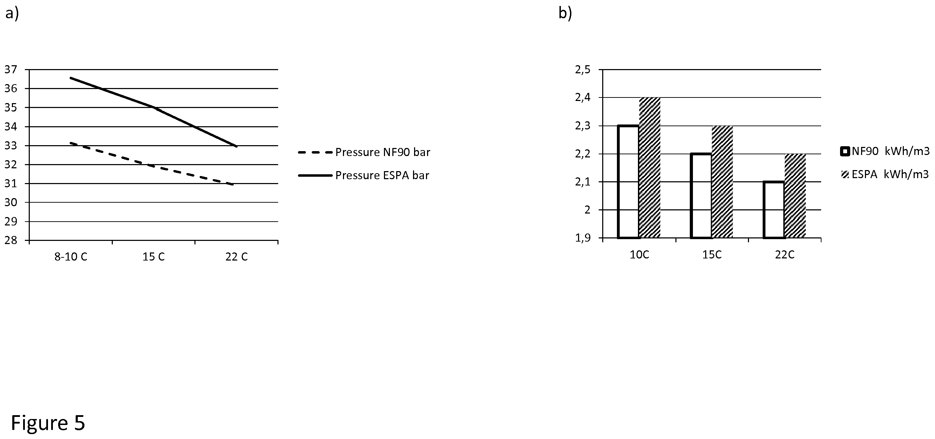

Figure 5 a) is diagram showing the hydrostatic pressure applied on the seawater (lumen side of the membranes) in nanofiltration membranes applied for producing softened seawater according to the invention, and Figure 5 b) presents the corresponding energy consumption per $m^3$ produced softened seawater.

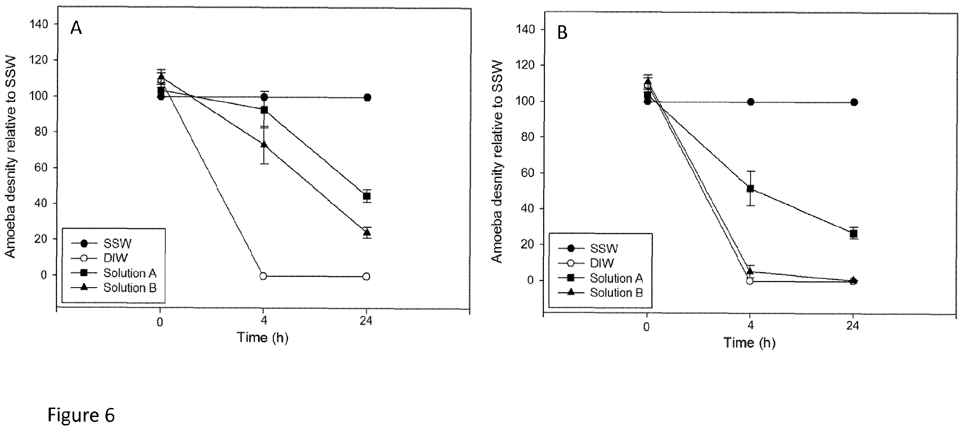

Figures 6 a) and 6 b) present comparative diagrams showing experimental results of exposing *Neoparamoeba perurans* to 34 g/kg NaCl in deionized water (solution A) and 17g/kg NaCl in deionized water (solution B) after 4 and 24 h. Figure 6 a) represents the total number of attached amoebae (polymorphic and rounded morphologies), whereas figure 6 b) represents only attached polymorphic amoebae. Error bars represent +/- 1 SEM.

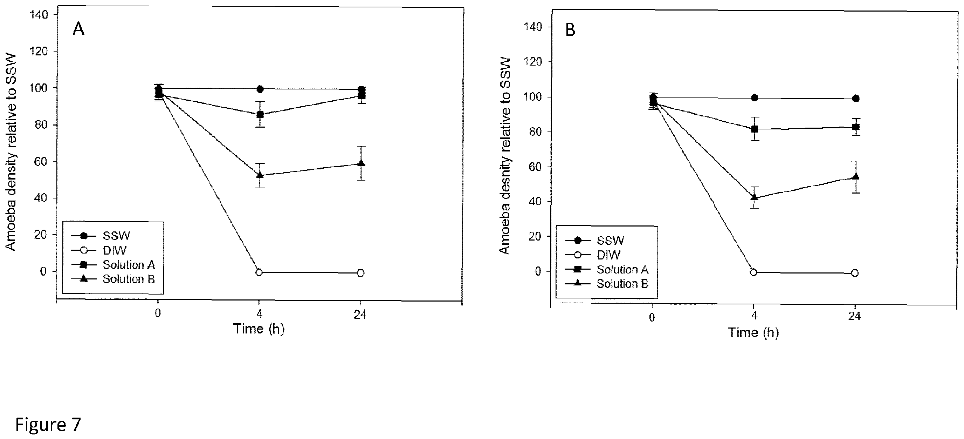

Figures 7 a) and 7 b) present comparative diagrams showing experimental results of exposing *Neoparamoeba perurans* to 34 g/kg NaCl and 500 mg/kg $CaCl_2$ in deionized water (solution A) and 17g/kg NaCl and 250 mg/kg $CaCl_2$ in deionized water (solution B) after 4 and 24 h. Figure 7 a) represents the total number of attached amoebae (polymorphic and rounded morphologies), whereas figure 7 b) represents only attached polymorphic amoebae. Error bars represent +/- 1 SEM.

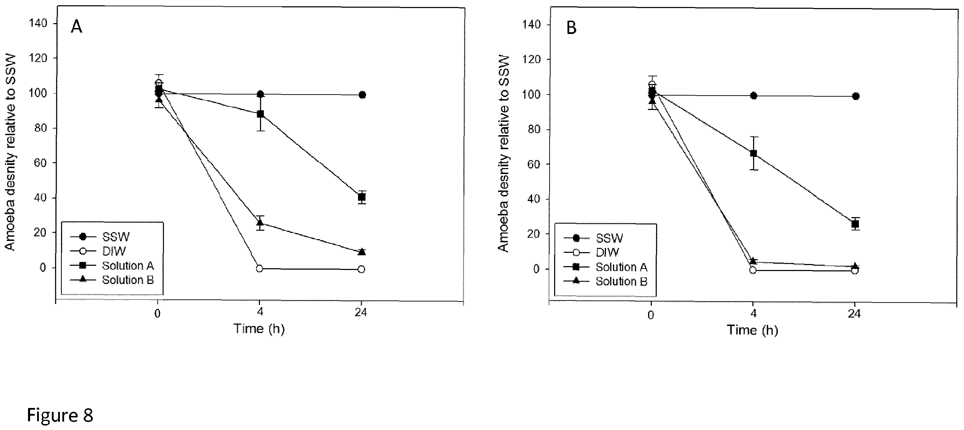

Figures 8 a) and 8 b) present comparative diagrams showing experimental results of exposing *Neoparamoeba perurans* to 34 g/kg NaCl and 500 mg/kg $MgCl_2$ in deionized water (solution A) and 17g/kg NaCl and 250 mg/kg $MgCl_2$ in deionized water (solution B) after 4 and 24 h. Figure 8 a) represents the total number of attached amoebae (polymorphic and rounded morphologies), whereas figure 8 b) represents only attached polymorphic amoebae. Error bars represent +/- 1 SEM.

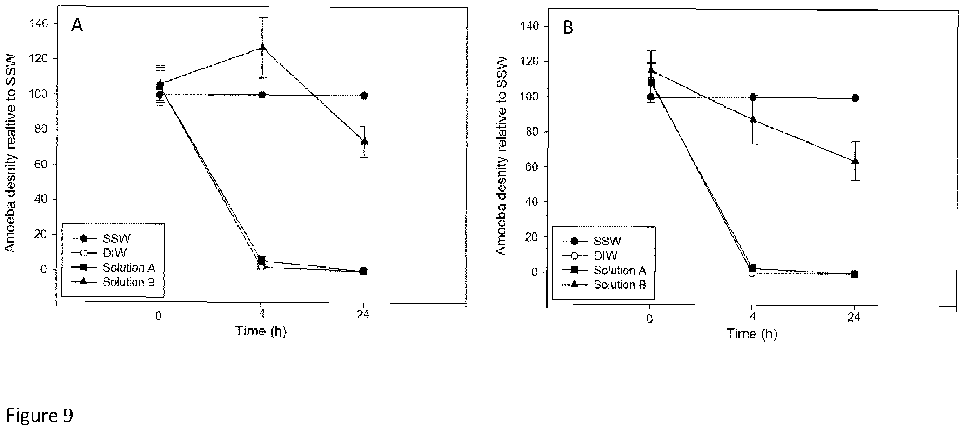

Figures 9 a) and 9 b) present diagrams showing experimental results of exposing *Neoparamoeba perurans* to softened seawater having a salinity of 4.2 g/kg and a $Ca^{2+}$-content of 3.1 mg/kg (Solution A) and comparative softened seawater having a salinity of 22 g/L and a $Ca^{2+}$ content of 180.5 mg/kg (Solution B) after 4 and 24 h. Figure 9 a) represents the total number of attached amoebae (polymorphic and rounded morphologies), whereas figure 9 b) represents only attached polymorphic amoebae. Error bars represent +/- 1 SEM.

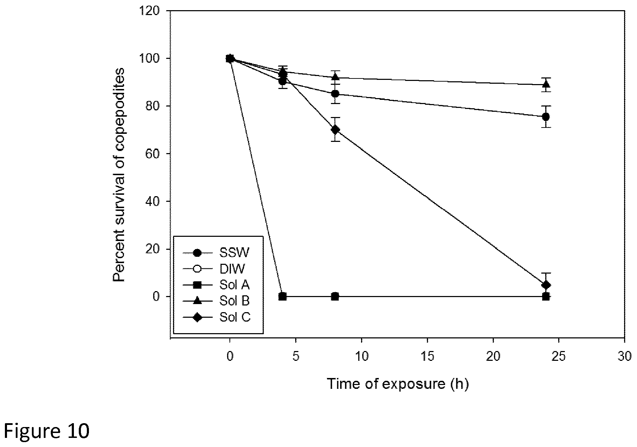

Figure10 present a diagram showing experimental results of exposing *Lepeophtheirus salmonis* (at all life-cycle stages) to softened seawater having a salinity of 4.2 g/kg and a $Ca^{2+}$ content of 3.1 mg/kg (Solution A), and to comparative softened seawater having a salinity of 22 g/kg and a $Ca^{2+}$ content of 180.5 mg/kg (Solution B), and to softened seawater having a salinity of 14.6 g/kg and a $Ca^{2+}$ content of 18.1 mg/kg (Solution C), after 4 and 24 h, respectively. Error bars represent +/- 1 SEM.

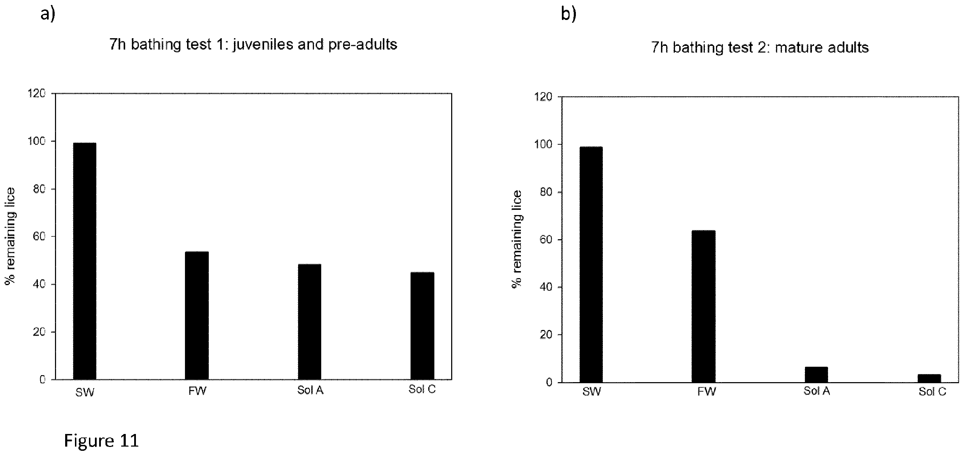

Figures 11a) and b) present a diagram showing experimental results of exposing *Lepeophtheirus salmonis* (at all life-cycle stages) *in vivo* on salmon swimming in softened seawater having a salinity of 4.2 g/kg and a $Ca^{2+}$ content of 3.1 mg/kg (Sol A) and softened seawater having a salinity of 14.6 g/kg and a $Ca^{2+}$-content of 18.1 mg/kg (Sol C) after 7 hours, as compared with seawater marked as "SW" and freshwater marked as "FW" on the figures. Figure 11a) presents the results on juveniles and pre-adults and figure 11b) presents the results on adult sea-lice.

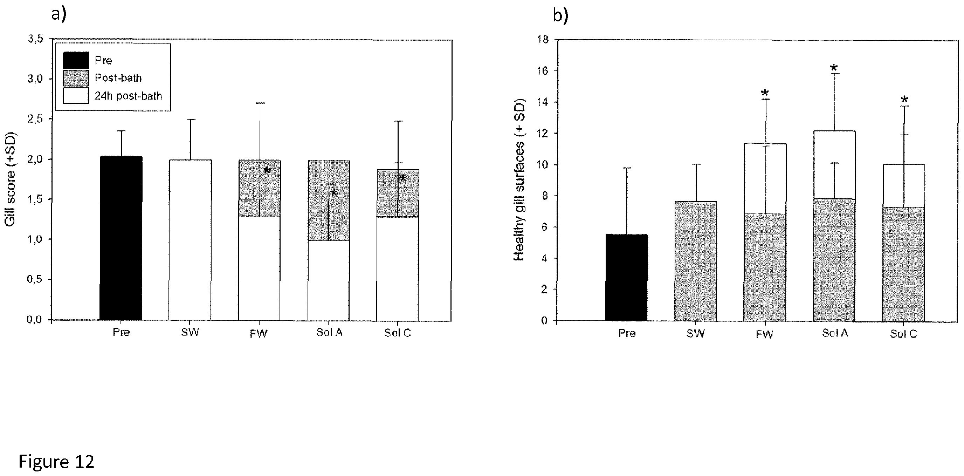

Figures 12 a) and b) present a diagram showing mean (+SD) gross gill score (fig. 12 a) and healthy (unaffected) gill surfaces (fig. 12 b) of Atlantic salmon smolts affected by amoebic gill disease, respectively. * represents significant difference from seawater (SW) control.

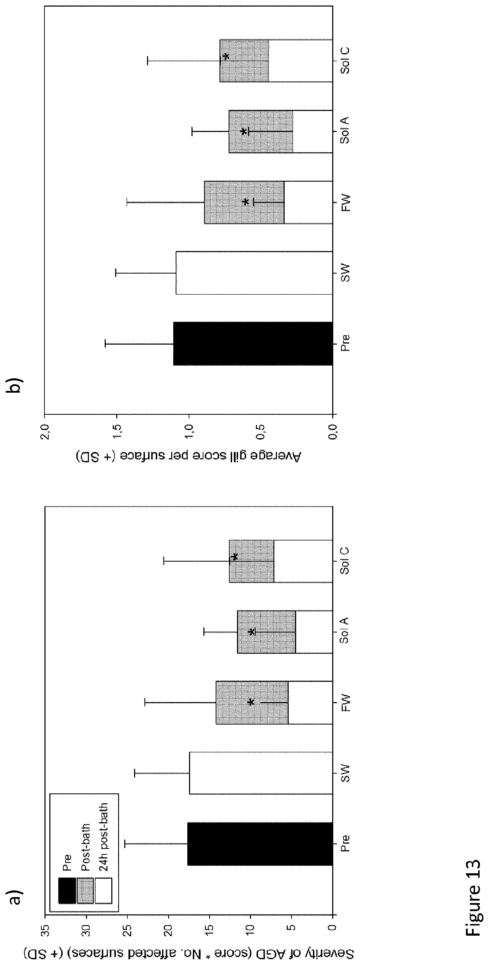

Figures 13 a) and b) present a diagram showing mean (+SD) severity of amoebic gill disease (fig. 13 a) and average gill score per surface (fig. 13 b) of Atlantic salmon smolts affected by amoebic gill disease, respectively. * represents significant difference from seawater (SW) controls.

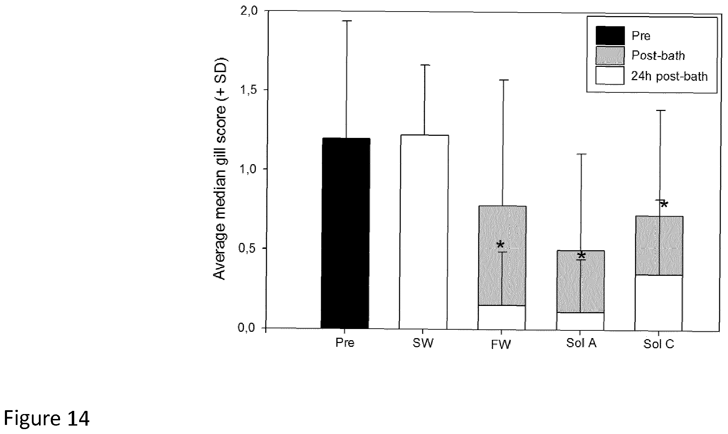

Figure 14 presents a diagram showing mean (+SD) median gill score of all gill surfaces of Atlantic salmon smolts affected by amoebic gill disease after being treated with freshwater, solution A, or solution C as compared with seawater. * represents significant difference from seawater (SW) controls.

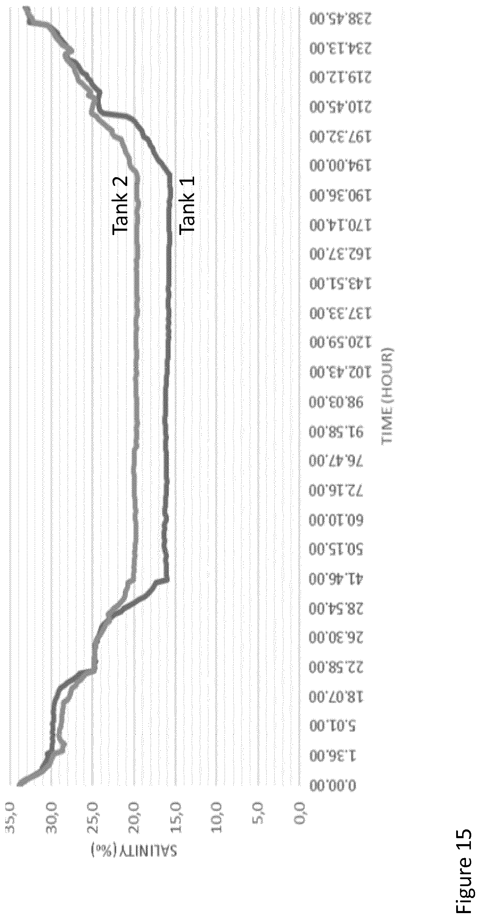

Figure 15 presents a diagram showing salinity profile of tanks 1 and 2 supplied with 15.9 and 19.9 ppt modified produced seawater, respectively.

## Example embodiment of the invention

The invention is further illustrated by way of an example embodiment.

An example embodiment of the system 1 according to the second aspect of the invention is shown schematically in figure 1, which is a cut-view as seen from the side. The system comprises a supply unit 2 for softened seawater and a treatment zone 3 in the form of a volume of softened seawater contained in the interior of a floating container 4 being partly submerged into the sea 5 by the aid of a floater 8.

The supply unit 2 is supplied with natural seawater by line 13, converts the natural seawater to softened seawater according to the invention, and then passes the softened seawater through line 6 to the treatment zone 3.

The walls of container 4 should be forming an enclosure isolating the interior space of the container 4 from the surrounding (natural) seawater 5 such that surrounding seawater cannot penetrate the treatment zone 3 in a significant degree. The container may be made of any water-resistant material having the mechanical strength and corrosion resistance to enable standing partly submerged in the sea and be subject to wave motions and weather phenomena usually encountered in marine environments.

The supply unit 2 of softened seawater supplies, either continuously or intermittently, softened seawater 6 into the treatment zone 3 at the interior of tank 4 through an inlet 7 located at the upper side of the container 4. In this way, the interior of container 4 becomes partly filled with the softened seawater which forms the treatment zone 3. The fishes 9 to be treated are contained into the volume of softened seawater constituting the treatment zone 3. Fish to be treated may be inserted into the treatment zone 3 and taken out of the treatment zone after treatment through a second closable inlet 10.

In this example embodiment, the supply unit 2 of softened seawater supplies a continuous flow of softened seawater, such that the container 4 needs an outlet 11 for excess softened seawater. The outlet 11 may advantageously have a grate 12 or other water penetrable closure preventing fishes 9 in the treatment zone 3 to escape into the surrounding sea 5. Due to the flow resistance through the grate 12, the hydrostatic pressure in the water in the treatment zone becomes somewhat higher that the hydrostatic pressure in the natural seawater outside the inlet 11 such that the surrounding seawater is prevented from flowing in through the outlet. This is indicated in figure 1 by non-filled arrows indicating the water level of the surrounding sea and the softened seawater of the treatment zone. The latter water level is somewhat higher. If the supply unit 2 supplies softened seawater intermittently, the outlet 11 may advantageously have a closable water-tight closure to prevent surrounding seawater 5 to penetrate the treatment zone 3.

Figure 2 illustrates an example embodiment of how the system according to the second aspect of the invention may be incorporated into a marine net-cage type fish farm having a net 14 enclosing a volume of natural seawater 5 for containing a number of fishes to be farmed 15. The net-cage is floating in the surface region of the sea by the aid of a floater 16.

The supply unit 2 may advantageously use one of more nanofiltration membrane module(s) for converting the natural seawater to softened seawater. The working principle of membrane based nanofiltration is shown schematically in figure 3. The figure illustrates a cylindrical housing 20 having an inlet 21 for injection of seawater located at one side of the housing 20. The inlet 21 is in fluid communication with the natural seawater line 13 and the outlet 26 for softened seawater is either passed to line 6 for being supplied to the treatment zone 3, or to the inlet 21 of a second nanofiltration membrane module if two or more nanofiltration membrane modules are assembled in series; as shown in figure 4.

The seawater being injected flow enters the lumen side 22 of a cylindrical hollow semipermeable membrane 23 as an inlet flow (indicated by arrow marked A on the figure). Part of the inlet flow A passes through the lumen side 22 of the hollow semipermeable membrane 23 and exits through an outlet 24 located at the opposite side of the housing 20 as a retentate flow (indicated by arrow B on the figure). The remaining part of the injected seawater is due to an applied hydrostatic pressure on the lumen side 22 forced through the hollow semipermeable membrane 23 and enters the compartment of the housing 20 on the exterior side 25 of the membrane 23 as a permeate flow (indicated by arrows C on the figure). The part of the seawater having passed through the membrane 23 and entered the compartment on the exterior side 25 exits the housing 20 through a second outlet 26 as a permeate flow ((indicated by arrow D on the figure). The hollow semipermeable membrane 23 divides the interior space of the cylindrical housing 20 into two compartments, one defined by the lumen side 22 and the other defined by the exterior side 25 of the membrane and the walls of the cylindrical housing 20.

One advantage of employing a nanofiltration membrane module to supply the softened seawater is that the modules may easily be arranged in parallel to meet any demand for softened seawater encountered in practice in fish farming. Another advantage of using a nanofiltration membrane is that since it does not remove all salt content, but allows a relatively large fraction of the monovalent salt ions to pass through the membrane and a relatively small fraction of the divalent salt ions, that the pressure drop over the membrane and thus energy consumption for producing the softened seawater is significantly less as compared to using reverse osmosis filtration to produce freshwater from seawater. Figures 5a) and 5 b) present energy consumption data for two commercial available nanofiltration membrane modules when producing softened seawater by filtrating natural seawater. The energy consumption is around 2 kWh/m$^3$ or less, which is less than about 1/3 of the typical energy consumption for making freshwater by filtrating natural seawater in a reverse osmosis membrane module.

Even though it is advantageous, and thus preferable, to apply a battery of M nanofiltration membrane modules assembled in series, i.e. that the natural seawater passes only once through a membrane module to be converted to the softened seawater according to the invention, it is envisioned that there may be applied a battery of M x N nanofiltration membrane modules, where M is number of modules assembled in parallel in each column and N is the number of modules assembled in series in each row. In practice, the number M may advantageously be any integer value from 1 to 100 and N may advantageously be an integer value selected from; 1, 2, 3, 4, or 5. An example embodiment of a battery of 4 x 2 nanofiltration membrane modules is shown schematically in figure 4. The example embodiment shown in figure 4 may produce a volume flow of softened seawater approx. four times the volume flow of softened seawater than obtainable with a single membrane module, and the softened natural seawater flowing through inlet 6 into the treatment zone 3 have been subject to two filtration steps. In this manner, it is possible to customise the calcium removal rate and production volume of the softened seawater to accommodate any conceivable need for softened seawater for treating fish in marine fish farms.

**Verification experiments production of softened seawater**

**(not according to the invention).**

A series of tests of softening seawater has been made with two commercially available nanofiltration membrane modules; Filmtec™ NF90-400 and Hydranautics ESPA 30G.

The Filmtec™ NF90-400 module has a polyamide thin-film composite membrane with a total surface of 37 m$^2$. The module may operate with a trans-membrane pressure up to a maximum of 41 bar, and be fed with seawater at a maximum feed flow of 15.9 m$^3$/hour. The Hydranautics ESPA3 OG module has a composite polyamide membrane with total surface area of 37.1 m$^2$. The module may operate be with a trans-membrane pressure up to a maximum of 41 bar and a maximum feed flow of 17.0 m$^3$/hour.

The tests were made with 16 samples of natural seawater taken at a depth of 17 meter outside Malm, a location in the fjord Trondheimsfjorden in Norway. The water in Trondheimsfjorden is oceanic Atlantic water mixed with a fraction of river water, and is thus somewhat less saline than the oceanic water. Otherwise, the fjord water is indistinguishable from the Atlantic oceanic water. The salinity and composition of the seawater samples are thus estimated by conversion of measured electrical conductivity of the samples under the assumption that the sample water has the same composition ratios as the Atlantic oceanic water. The resulting compositions are presented in table 1.

As shown in Table 1, the seawater samples had a total salinity in the order of 29 - 30 g/L, and a $Ca^{2+}$-content of around 320 - 350 mg/L. The density of saltwater of salinity 30 g/L and temperature in the range from 5 to 20 °C is in the range of 1020 to 1023 $kg/m^3$, such that the "g/L" unit is almost similar to the "g/kg" unit as specified in the appended claims. Eight seawater samples were passed through each of the above presented membrane modules. Figure 5 a) presents the hydrostatic pressure applied on the seawater (lumen side of the membranes), and figure 5 b) presents the corresponding energy consumption per $m^3$ permeate (produced softened seawater). Both applied membrane modules may provide a permeate flow up to about 3 $m^3$/hour.

Table 2 and 3 below presents the measured calcium and total dissolved salt contents in the permeate obtained with the Hydranautics ESPA3 OG and the Filmtec™ NF90-400 membrane module, respectively.

The figures in tables 1 to 3 corresponds to rejection rates of $Na^+$ in the seawater samples in the order of 70 - 90 %, corresponding to a salinity of the permeate flow from the membrane module from 2.3 to 6.9 g/kg. The calcium rejection rates are in the range of 99.0 to 99.6 %, corresponding to a $Ca^{2+}$ content from 1.2 to 3.2 mg/kg. These values are well within the specification of the softened seawater for use in the present invention.

Table 1 Measured conductivity in seawater samples applied in the tests.

| Sample id. | Measured cond. [μS/cm] | Estimated concentrations | | | | | | |
|---|---|---|---|---|---|---|---|---|
| | | TDS, [ppm] | TDS, [mg/L] | $Ca^{2+}$ [mg/L] | $Mg^{2+}$ [mg/L] | $SO_4^{2-}$ [mg/L] | $Na^+$ [mg/L] | $Cl^-$ [mg/L] |
| PV-1A | 42700 | 27454 | 26890 | 315 | 983 | 2076 | 8254 | 14812 |
| PV-2A | 43100 | 27738 | 27168 | 319 | 993 | 2097 | 8339 | 14965 |
| PV-3A | 44550 | 28772 | 28180 | 331 | 1030 | 2176 | 8650 | 15523 |
| PV-4A | 44300 | 28593 | 28005 | 329 | 1023 | 2162 | 8596 | 15427 |
| PV-5A | 44750 | 28915 | 28320 | 332 | 1035 | 2186 | 8693 | 15600 |
| PV-6A | 44800 | 28951 | 28356 | 333 | 1036 | 2189 | 8704 | 15619 |
| PV-7A | 45400 | 29381 | 28777 | 338 | 1051 | 2222 | 8833 | 15852 |
| PV-8B | 46700 | 30317 | 29693 | 348 | 1085 | 2292 | 9114 | 16356 |
| PV-9B | 45700 | 29597 | 28988 | 340 | 1059 | 2238 | 8898 | 15968 |
| PV-10B | 46000 | 29812 | 29199 | 343 | 1067 | 2254 | 8963 | 16084 |
| PV-11B | 46100 | 29884 | 29270 | 343 | 1069 | 2260 | 8984 | 16123 |
| PV-12B | 46500 | 30173 | 29552 | 347 | 1080 | 2281 | 9071 | 16279 |
| PV-13B | 46900 | 30461 | 29835 | 350 | 1090 | 2303 | 9158 | 16434 |
| PV-14B | 46700 | 30317 | 29693 | 348 | 1085 | 2292 | 9114 | 16356 |
| PV-15B | 46650 | 30281 | 29658 | 348 | 1084 | 2290 | 9103 | 16337 |

Table 2 Results from analyses of softened seawater made by the Hydranautics ESPA 30G membrane.

| Sample | Conductivity [mS/cm] | Temperature [°C] | TDS [mg/L] | $Ca^{2+}$ [μg/L] |
|---|---|---|---|---|
| PV-8B | 5.68 | 8.6 | 2840 | 1222 |
| PV-9B | 5.52 | 8.3 | 2800 | 1243 |
| PV-10B | 7.71 | 14.9 | 2330 | 1730 |
| PV-11B | 7.70 | 15.2 | 3840 | 1674 |
| PV-12B | 10.00 | 21.7 | 5020 | 2349 |
| PV-13B | 9.23 | 21.0 | 4610 | 2213 |
| PV-14B | 9.77 | 21.4 | 4900 | 2532 |
| PV-15B | 9.28 | 21.2 | 4640 | 2125 |

Table 3 Results from analyses of softened seawater made by the Filmtec™ NF90-400 membrane.

| Sample | Conductivity [mS/cm] | Temperature [°C] | TDS [mg/L] | $Ca^{2+}$ [µg/L] |
|---|---|---|---|---|
| PV-1A | n.a. | 10.0 | 4780 | 2596 |
| PV-2A | 9.32 | 10.0 | 4680 | 2742 |
| PV-3A | n.a. | n.a. | n.a. | 2293 |
| PV-4A | n.a. | 15.0 | 5400 | 2731 |
| PV-5A | 11.15 | 14.8 | 5670 | 3114 |
| PV-6A | n.a. | 22.0 | 6950 | 3181 |
| PV-7A | 12.82 | 22.0 | n.a. | 2597 |
| PV-8A | n.a. | 23.0 | n.a. | 2827 |
| **n.a. - not analysed/measured** | | | | |

**Experimental results from tests on killing rates of *Neoparamoeba perurans* in vitro**

Experiment 1 (comparative)

Amoeba cultures of the ES301013 C2 clone were cultured at 15 °C in cell culture flasks over layered with Malt-Yeast broth MYB: (0.1 g/L malt extract, 0.1 g/L yeast extract in sterile seawater). Cultures were divided weekly to maintain optimal cell growth.

Cultures of optimal densities (approximately 40000 cells/mL) were isolated by scraping and the suspension of cells aliquoted into a 24 well cell culture plate giving an approximate density of 3000 or 5000 cells per well. Fresh MYB was added to make a starting volume of 1 mL per well.

After 24h, the density of attached amoeboid cells were counted on 5 non-contiguous fields per well. The liquid supernatant was then replaced in each series of 6 wells per plate and replaced with 1 mL of either:

- Sterile autoclaved seawater having a salinity of 34.4 g/L [33.4 g/kg], of which: > 5000 mg/L [> 4860 mg/kg] is $Na^+$, 396 mg/L [384 mg/kg] is $K^+$, 1180 mg/L [1146 mg/kg] is $Mg^{2+}$, 418 mg/L [406 mg/kg] is $Ca^{2+}$, and 18800 mg/L [18270 mg/kg] is $Cl^-$, for control treatment allowing assessment of positive growth of amoebae.

- Deionised freshwater having a salinity of less than 0.01 g/L [less than 0.0097 g/kg], of which: 6.2 mg/L [6.0 mg/kg] is $Na^+$, 0.27 mg/L [0.26 mg/kg] is $K^+$, < 0.1 mg/L [< 0.097 mg/kg] is $Mg^{2+}$, < 0.1 mg/L [< 0,097 mg/kg] is $Ca^{2+}$, and 0.05 mg/L [0.049 mg/kg] is $Cl^-$ for negative control ensuring all amoebae were non-viable or attached to the well surface.

- Comparative treatment solution A: Dissolving 34 g/kg NaCl in deionised freshwater

- Comparative treatment solution B: Dissolving 17 g/kg NaCl in deionised freshwater.

The conversion from mg/L to mg/kg herein is, unless specified otherwise, calculated for seawater having a density of 1029 kg/m$^3$.

All plates were sealed with parafilm tape. Each assay was performed in duplicate (2 plates) with the row order of the treatments randomly assigned per plate.

After 4 h the number of attached amoebae, showing normal polymorphic amoeboid morphology and those showing a rounded morphology were counted in 5 non-contiguous fields of view from each well on each plate. This was repeated after 24h of exposure. All assays were performed in duplicate with 6 wells per assay. The amoeba density was then expressed are proportional to the sterile seawater control (SSW) to allow comparison between assays and plates (accounting for small variations in initial amoeba numbers per well, plate or assay).

Figures 6 a) and 6 b) show the effects of exposure of *Neoparamoeba perurans* to 34 g/kg NaCl in deionized water (solution A) and 17g/kg NaCl in deionized water (solution B) after 4 and 24 h, respectively. Figure 6 a) represents the total number of attached amoebae (polymorphic and rounded morphologies), whereas figure 6 b) represents only attached polymorphic amoebae. Error bars represent +/- 1 SEM. The sterile autoclaved seawater is marked as "SSW", the deionised freshwater is marked as "DIW", solution A is marked as "Solution A" and solution B is marked as "Solution B" in the figures.

As seen from figures 6 a) and 6 b), deionised freshwater supplemented with only NaCl had a marked killing effect over 24 h with a reduction in the total amoebae numbers attached to the plate and the number of polymorphic amoebae visible. Over a shorter exposure duration (4h) the total number of attached amoebae was marginally reduced under these conditions, although the number of polymorphic amoebae was substantially reduced. Under more dilute conditions (solution B), amoeba attachment was more substantially reduced. This result indicates that water having high salt contents at levels found in natural seawater may be toxic to the amoeba where other ions such as $Ca^{2+}$ and $Mg^{2+}$ are absent.

Experiment 2 (comparative)

The same experimental procedure as described for experiment 1 was repeated, but now with a comparative treatment solution A made by dissolving 34 g/kg NaCl and 500 mg/kg $CaCl_2$ in deionised freshwater, resulting in a $Ca^{2+}$ content of 180.5 mg/kg, and a comparative treatment solution B was made by diluting solution A by 50 % with deionized freshwater to achieve a content of 17 g/kg NaCl and 250 mg/kg $CaCl_2$, resulting in a $Ca^{2+}$ content of 90.3 mg/kg.

The effect of exposing *Neoparamoeba perurans* to solution A and solution B of experiment 2 is shown in Fig. 7 a) and 7 b), respectively. Fig. 7 a) represents the total number of attached amoebae (polymorphic and rounded morphologies), whereas fig. 7 b) represents only attached polymorphic amoebae. Error bars represent +/- 1 SEM. The sterile autoclaved seawater is marked as "SSW", the deionised freshwater is marked as "DIW", solution A is marked as "Solution A" and solution B is marked as "Solution B" in the figures.

These figures show that the exposure to the water having NaCl and somewhat less calcium than natural seawater reduced the number of attached amoeba after 4 hours, but that the amoeba managed to tolerate the salinity and recover to a certain extent after 24 hours of exposure. This result indicates that when the water contains salt and calcium, the amoeba has a higher survival.

A comparison of figures 6 a) and b) with figures 7 a) and b) show further that there was a significantly reduced loss of attached amoebae when exposed to the artificially made seawater containing both $Na^+$ and $Ca^{2+}$ as compared to the water having only $Na^+$, both in terms of total amoeba density and polymorphic amoebae. This effect was most profound for solution A having the highest calcium content (180.5 mg/kg $Ca^{2+}$). This result indicates that it is not the salt content as such that is important, but the presence of calcium.

Experiment 3 (comparative)

The same experimental procedure as described for experiment 1 was repeated, but now with a comparative treatment solution A made by dissolving 34 g/kg NaCl and 500 mg/kg $MgCl_2$ in deionised freshwater, resulting in a $Mg^{2+}$-concentration of 130 mg/kg, and a comparative treatment solution B was made by diluting solution A by 50 % with deionized freshwater to achieve a content of 17 g/kg NaCl and 250 mg/kg $MgCl_2$, resulting in a $Mg^{2+}$-content of 65 mg/kg.

The effect of exposing *Neoparamoeba* perurans to solution A and solution B of experiment 3 is shown in Fig. 8 a) and 8 b), respectively. Fig. 8 a) represents the total number of attached amoebae (polymorphic and rounded morphologies), whereas fig. 8 b) represents only attached polymorphic amoebae. Error bars represent +/- 1 SEM. The sterile autoclaved seawater is marked as "SSW", the deionised freshwater is marked as "DIW", solution A is marked as "Solution A" and solution B is marked as "Solution B" in the figures.

Figures 8 a) and 8 b) show that the number of attached amoeba is reduced similarly as in experiment 1. The amoeba does not manage to adopt to the water having $Na^+$ and $Mg^{2+}$. The number of attached amoeba after 24 hours of exposure is considerably smaller than after 4 hours of exposure. This result is consistent with the result of experiment 2, that it is not the salt content as such that is important, but the presence of calcium seems to be essential.

Experiment 4

The same experimental procedure as described for experiment 1 above was repeated with sterilized and filtrated natural seawater taken from Trondheimsfjorden outside of Vanvikan, in Norway.

Solution A was made by nanofiltering the sterilized seawater in a Filmtec™ NF90-400 membrane module providing softened seawater having a salinity of 4.3 g/L [4.2 g/kg] of which 1450 mg/L [1409 mg/kg] is $Na^+$, 64.5 mg/L [62.7 mg/kg] is $K^+$, 7.2 mg/L [7.0 mg/kg] is $Mg^{2+}$, 3.29 mg/L [3.20 mg/kg] is $Ca^{2+}$, and 2470 mg/L [2400 mg/kg] is $Cl^-$.

Comparative solution B was made by filtering the sterilized seawater in a Hydranautics NanoSW membrane module providing softened seawater having a salinity of 22.6 g/L [22.0 g/kg] of which > 5000 mg/L [> 4860 mg/kg] is $Na^+$, 286 mg/L [278 mg/kg] is $K^+$, 123 mg/L [119.5 mg/kg] is $Mg^{2+}$, 122 mg/L [118.5 mg/kg] is $Ca^{2+}$, and 12600 mg/L [12244 mg/kg] is $Cl^-$.

The effect of exposing *Neoparamoeba perurans* to the seawater of solution A and solution B above is shown in figure 9 a) and 9 b), respectively as compared to exposing the amoeba to sterilized seawater and deionized freshwater (the latter two solutions having similar compositions as given above for experiment 1). Solution A showed a significant reduction in both the total number of amoebae and the number of polymorphic amoebae comparable to that of deionized water (Fig. 9 a). Solution B (fig 9 b), however, reduced the number of attached amoebae only after 24 h exposure. Fig. 9 a) represents the total number of attached amoebae (polymorphic and rounded morphologies), whereas fig. 9 b) represents only attached polymorphic amoebae. Error bars represent +/- 1 SEM. The sterile autoclaved seawater is marked as "SSW", the deionised freshwater is marked as "DIW", solution A is marked as "Solution A" and solution B is marked as "Solution B" in the figures.

The result of experiment 4 demonstrates that softened seawater having a salinity and calcium content according to the present invention has a profound killing effect on *Neoparamoeba perurans*. The effect is comparable to the killing effect of freshwater.

**Experimental results from tests on killing rates of *Lepeophtheirus salmonis copepodites* (the infective stage of the salmon louse).**

Experiment 5

Copepodites produced by ILAB (Bergen, Norway) were used in this study. 7-22 copepodites (range) were incubated in a 24 well flat-bottomed culture plate containing either sterile seawater (SSW), deionized water (DIW), or the same solution A or Comparative solution B as used in experiment 4, and a solution C having a salinity of 15 g/L [14.6 g/kg] of which > 5000 mg/L [> 4860 mg/kg] is $Na^+$, 196 mg/L [190 mg/kg] is $K^+$, 15.9 mg/L [15.5 mg/kg] is $Mg^{2+}$, and 18.6 mg/L [18.1 mg/kg] is $Ca^{2+}$. The chloride ion content was not measured for solution C. Thus, solution A had a salinity of 4.2 g/kg and a $Ca^{2+}$ content of approx. 3 mg/kg, solution B had a salinity of 22 g/kg and a calcium content of approx. 180 mg/kg, and solution C had a salinity of 14.6 g/kg and a $Ca^{2+}$ content of approx. 18 mg/kg. Solution C was obtained by mixing an amount of solution A and softened seawater made by a GE DK-400 nanofiltration membrane module.

The plate was incubated at 15 °C for 24 h. The number of live and dead copepodites was counted visually after 4, 8 and 24 h exposure to the test solutions. All experiments were performed in triplicate plates with 6 wells per treatment. The result of the test is shown in Fig. 10. As seen in the figure, there was good survival of copepodites in the SSW treatment with over 80 % survival over 24 h. Treatment with DIW and solution A resulted in 100 % mortality within 4 h comparable with that of deionized freshwater. Solution C also had a profound and strong killing effect on the sea-louse, while solution B which had a high content of calcium showed no killing effect above the sterilized seawater showing good survival over 90% over the 24 h exposure period.

The result of experiment 5 demonstrates that softened seawater having a salinity and calcium content according to the present invention has a profound killing effect on sea-louse at the copepodite stage. The effect is comparable to the killing effect of freshwater.

Experiment 6

In this experiment, the removal effect of the softened seawater on sea-louse *in vivo,* i.e. on living specimens of salmon is investigated.

The experiment applied the same control of seawater and fresh water similar to that in experiments 1 to 5 above (albeit natural, i.e. non-sterile and non-deionized seawater and freshwater, respectively), and the same solution A and solution C as given for experiment 5 above.

The experiment was performed as follows:

A population of Atlantic salmon post-smolts (approximate size 130g) were maintained in a 3000 L tank with flowing filtered seawater (salinity of 34 g/kg) at 8.0 °C. Fish were hand fed daily with pelleted commercial feed. The flow to the tank was stopped for 1 hour and copepodites added to the tank approximately 30-50 copepodites per fish. The fish were then maintained for a further 2 weeks, inspected for the presence of sea lice, then an additional repeat challenge of 20 copepodites per fish was made. After a further 3 weeks, fish were used in bathing trials as described below.

Challenge test 1: 30 Atlantic salmon with a mix of immature adult and juvenile sea lice were anaesthetized with MS-222 (100 mg/L) and the number of pre-adult and juvenile lice evaluated on each fish. Fish were then recovered in a 200 L stainless steel tank containing 126 L of either aerated sea water, freshwater, Solution A or Solution C. Fish were maintained for 7 h, then netted, anaesthetized with MS-222 and re-examined for lice. Fish were recovered and returned to the main infection tank.

Challenge test 2: After a further 3 weeks of sea lice infestation, 30 Atlantic salmon were anaesthetized with MS-222 (100 mg/L) and the number of adult lice counted on each fish. Fish were then recovered in a 200 L stainless steel tank containing 126 L of either aerated sea water, freshwater, Solution A or Solution C. Fish were maintained for 7 h, then netted, anaesthetized with MS-222 and re-examined for lice.

Figure 11 a) is a diagram presenting the removal rate on juveniles and pre-adults after 7 hours of exposure. Figure 11 b) show a similar effect for adult sea-louse. The experiments include a comparison test with natural seawater, marked as "SW" on the figures and natural freshwater, marked as "FW" on the figures.

As seen on the figures, the softened seawater has a clear and profound removal effect on sea-louse living on a host when the host is contained in the water for a period of a few hours, especially for adult sea-louse. Moreover, those lice surviving on fish even after treatment with modified water were all male lice and all gravid females had been removed during the test. Here the test gave a far better removal effect on the adult sea-louse than the freshwater treatment.

Conclusion of experimental results

In conclusion, the selective removal of calcium ions in addition to reduced salinity (brackish water) enhances to the effects on detaching *Neoparamoeba perurans* from culture surfaces, leading to a stressing of the cells (formation of rounded morphologies). Similarly, dilute environments of brackish water with reduced Ca also have an enhanced killing effect upon *Lepeophtheirus salmonis* in the copepodite and adult life cycle stages and removal of lice from the skin surfaces of infected fish.

**Experimental results from tests on killing rates of *Neoparamoeba perurans* in vivo**

Experiment 7

103 Atlantic salmon post-smolts raised at ILAB, Bergen were infected with *Neoparamoeba perurans* and maintained at 13 °C until characteristic gill patches developed indicating the likely presence of amoebic gill disease. Pre-screening of the fish prior to bath challenge, showed a gill score of an average of approximately 2; which is the clinical threshold at which many gill treatments for AGD occur within the aquaculture industry.

The population of AGD-affected fish was then distributed into one of 4 tanks containing 200 L of either comparative filtered seawater (ambient salinity 34 ppt, 13 °C); comparative deionized freshwater (ambient salinity 0 ppt); and the same solution A (salinity 4.4 ppt) or solution C (salinity 15 ppt) as given for experiment 5 above. Fish were maintained under static water conditions with supplemental air and oxygen to maintain oxygen levels above 80% saturation for a period 4 h.

At the end of the bathing period, 9 fish from each tank were removed, sedated and euthanized with 100 mg L$^{-1}$ MS222 (tricaine methane sulphonate). The fish were scored as follows:

1. Gross gill score (0-5 modified from Taylor et al. 2009, Table 4.) - an industry standard gill scoring system used in Norway scoring the worse affected gill arch.

2. The score 0-5 of each of the 16 gill surfaces

For all tanks, the tank level was then lowered to approximately 100 L and rapidly refilled with ambient seawater. Fish were maintained for 24 h, after which the remaining 9 fish form each tank were removed, sedated and scored as above.

*Results*

There was a significant reduction in gill score, and increase in the number of unaffected surfaces 24 h after the treatment bath period with freshwater, or solution A or C as shown in Fig. 12. Similarly, there was a significant decrease in the severity of the gill score and average score per gill surface 24 h after bath treatment with freshwater or solution A or C compared with seawater controls, as shown in Fig. 13. Seawater controls did not show any significant reduction in any of the gill scores 24 h post bath compared with pre-bath levels or immediately following the bath.

Table 4: Descriptive and numeric scores corresponding to non-specific gill lesioning and AGD pathology, adapted from Taylor et al. (2009).

| Infection level | AGD score | Description |
|---|---|---|
| Clear | 0 | No sign of infection |

(continued)

| Infection level | AGD score | Description |
|---|---|---|
| Very light | 1 | 1 white spot, light scarring or undefined necrotic streaking |
| Light | 2 | 2 - 3 spots/small mucus patches |
| Moderate | 3 | Established thick mucus patch or spot groupings up to 20 % of the gill |
| Advanced | 4 | Established lesions covering up to 50 % of gill area |
| Heavy | 5 | More than 50 % of gill areal covered |

There was a significant reduction in the median gill score for all surfaces in fish 24 h after being treated with freshwater, solution A or solution C compared with seawater (Fig. 14).

*Conclusion*

Bathing AGD-affected Atlantic salmon smolts under laboratory conditions showed a significant reduction in gill score of affected fish compared with the seawater controls. The reductions observed with modified seawater solutions A and C were equivalent to those achieved by freshwater - the current industry standard method for treatment.

Experiment 8 (comparative)

Amoebic gill disease caused by the parasitic amoebae *Neoparmaoeba perurans* in ballan wrasse (*Labrus bergylta*) is a common problem for the producers of cleanerfish in Norway. This parasite is also a pathogen of Atlantic salmon (*Salmo salar*) and thus the use of cleanerfish for control of sealice in Atlantic salmon production poses a significant risk for disease transmission between wrasse and salmon.

The primary method of disease control in wrasse is to reduce water salinity by the addition of freshwater to a salinity of 15 ppt and maintain fish during that period for at least 5-7 days before returning the salinity to normal seawater levels. This causes osmotic stress to the wrasse that are intolerant of a fully freshwater environment and salinities below 15 ppt. The aim of this study was to test if modified seawater produced by nanofiltration to lower the calcium and magnesium content of the water could be effective at controlling amoebic gill disease.

*Materials and methods*

Ballan wrasse (*Labrus bergylta*) juveniles of average mass 27.1 g were held in two tanks at a commercial hatchery facility in two 900 L tanks at a stocking density of 40.1 and 32.4 kg m$^{-3}$ respectively. Fish were diagnosed as having amoebic gill disease as determined by commercial qPCR.

The salinity of both tanks was initially 33 ppt, then, with the introduction of produced water gradually reduced to 15.9 g/kg (tank 1) and 19.9 g/kg (tank 2) over 42 h (see Table 5). The salinity was maintained at these levels until 195h after which the salinity was raised in both tanks to 34 ppt over a 42h period (Fig 1.). The prevalence of PCR positive fish were tested using fisher-exact test of chi-square analysis.

*Table 5. Water chemistry of the test water after 48 h of introduction into each of tank 1 or 2 respectively. Metal ion analysis performed by ICP-MS.*

| Parameter | units | Tank 1 | Tank 2 |
|---|---|---|---|
| pH | | 7.0 | 6.8 |
| Electric conductivity | mSm$^{-1}$ | 2620 | 3320 |
| Salinity | g/kg | 15.9 | 19.9 |
| Cl | mg L$^{-1}$ | 9180 | 11400 |
| Al | $\mu$g L$^{-1}$ | < 50 | < 50 |
| Fe | $\mu$g L$^{-1}$ | 106 | < 100 |

(continued)

| Parameter | units | Tank 1 | Tank 2 |
|---|---|---|---|
| Cu | $\mu$g L$^{-1}$ | < 10 | < 10 |
| Na | mg L$^{-1}$ | 5740 | 7010 |
| K | mg L$^{-1}$ | 221 | 271 |
| Ca | mg L$^{-1}$ | 27.4 | 35.4 |
| Mg | mg L$^{-1}$ | 24.4 | 39.6 |

Samples of 10 fish were removed from each tank by dip net prior to reducing the salinity, after 48 h (at test salinity) 195 h (before salinity was increased and after salinity had increased to 34 g/kg. Gill samples were collected for commercial qPCR test (Patogen) for *Neoparamoeba perurans,* the causative agent of amoebic gill disease.

*Results and discussion*

Initially 90 % of the fish in the test tested positive for *Neoparamoeba perurans,* the causative agent of amoebic gill disease in ballan wrasse. After the initial 48h of the study, the reduction from 90 % to 20 % or 4 0% prevalence of *Neoparamoeba* positive fish was not significant (Tank 1: Fisher exact P=0.128; tank 2: $X^2$ = 0.535, df = 1 P value = 0.464). However, over the duration of the study to 95 h exposure, there was a significant reduction in the prevalence of *Neoparamoeba peruans* positive fish (Tank 1: $X^2$ = 8.381 df =2, P value 0.015; tank 2: $X^2$ = 6.997 df = 2, P value = 0.03). It is noteworthy that after 238 h when the weater salinity was returned to 34 ppt, tank 2 had one fish testing positive for *Neoparamoenba perurans* (see Table 6).

These results indicate that exposure of ballan wrasse, clinically diagnosed with AGD according to the presence of *Neoparamoeba perurans* on the gills, to modified seawater with reduced levels of calcium and magnesium was sufficient to eliminate the presence of gill amoebae after 95 h. This effect was most marked when using the 16 ppt produced water.

*Table 6. Results of qPCR analysis offish gills sampled prior to (0 h), after salinity reduction (48h), after a period of reduced salinity (95 h) and upon return of salinity to 34g/kg (238 h).*

| Exposure time [hours] | Proportion PCR +ve Tank 1, 15.9 ppt | Proportion PCR +ve Tank 2, 19.9 ppt |
|---|---|---|
| 0 | 9/10 | |
| 48 | 2/10 | 4/10 |
| 95 | 0/10 | 0/10 |
| 238 | 0/10 | 1/10 |
| * Proportion of PCR +ve is proportion of fish what were positive for *Neoparamoeba perurans* infection based upon detection of their RNA. | | |

## References

1. Powell and Kristensen, 2014, "Freshwater treatment of amoebic gill disease and sea-lice in seawater salmon production; considerations of water chemistry and fish welfare.", NIVA, Report No. 6632-2014.

2. http://www.imr.no/nyhetsarkiv/2016/juli/ferskvatn_drep_best_unge_lakselus /nb-no

3. Lima et al., 2015, "Involvement of contractile vacuoles in the osmoregulation process of the marine parasitic amoeba Neoparamoeba perurans", Journal of Fish Diseases, DOI: 10.1111/jfd.12408.

4. Masterton and Slowinski (ed.) 1977, "Chemical Principles", W.B. Saunders Company, ISBN 0-7216-6173-4, page 299.

5. Taylor RS, Muller WJ, Cook MT, Kube PD, Elliott NG. 2009, Gill observations in Atlantic salmon (Salmo salar, L.) during repeated amoebic gill disease (AGD) field exposure and survival challenge. Aquaculture 290(1-2): 1-8. DOI: http://dx.doi.org/10.1016/j.aquaculture.2009.01.030

6. Timothy J. Green (2003), "Exploitation of Water Chemistry in the Treatment of Amoebic Gill Desease", Master thesis, School of Agriculture, University of Tasmania, October 2003, chapter 4.2.1.3.

7. Mark D. Powell et al. (2003), "In vitro survival and the effect of water chemistry and oxidative chemical treatments on isolated gill amoeba from AGD-affected Atlantic salmon", AQUACULTURE, (2003-04-14), pages 135-144, XP009506696.

8. Harriet Perry et al. (2001) "Calcium concentration in seawater and exoskeletal calcification in the blue crab, Callinectes sapidus", Aquaculture, Vol. 198, no. 3-4, pp. 197-208, XP55760245, DOI: 10.1016/S0044-8486(00)00603-7.

9. Yuefei Song et al. (2011), "Performance of UF NF integrated membrane process for seawater softening", DESALINATION, vol. 276, no. 1, pages 109-116, XP028230859, DOI: 10.1016/J.DESAL.2011.03.064.

**Claims**

1. A softened seawater for use as a medicament,
**characterised in that** the softened seawater has:

   - a salinity in the range from 1.0 to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
   - a $Ca^{2+}$ content of $\leq$ 100 mg/kg, determined as the mass of $Ca^{2+}$ ions in a sample of one kg of the softened seawater,

   and **in that** the softened seawater is used as medicament for reducing calcium sensitive ectoparasites on a marine fish, preferably one or more of: *Neoparamoeba perurans* or *Lepeophtheirus salmonis.*

2. A softened seawater for use in a method for treating marine fish for calcium sensitive ectoparasites by containing the fish for a period of time in the softened seawater, wherein the fish to be treated is a salmonid, preferably Atlantic salmon (*Salmo salar*) and the method is aimed at reducing *Neoparamoeba perurans* or *Lepeophtheirus salmonis,* and wherein the softened seawater has:

   - a salinity in the range from 1.0 to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
   - a $Ca^{2+}$ content of $\leq$ 100 mg/kg, determined as the mass of $Ca^{2+}$ ions in a sample of one kg of the softened seawater.

3. The softened seawater for use according to any of claims 1 to 2, wherein:

   - the salinity of the softened seawater is in one of the following ranges; from 1 g/kg to 12.5 g/kg, from 1 g/kg to 10 g/kg, from 2 g/kg to 15 g/kg, from 2 g/kg to 12.5 g/kg, or from 2 g/kg to 10 g/kg, or most preferably from 2 g/kg to 8 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
   - the $Ca^{2+}$ content of the softened seawater is in one of the following ranges; from 0.001 to 95 mg/kg, from 0.001 to 90 mg/kg, from 0.001 to 80 mg/kg, from 0.001 to 50 mg/kg, from 0.001 to 10 mg/kg, from 0.001 to 8 mg/kg, from 0.001 to 6 mg/kg, from 0.001 to 5 mg/kg, from 0.001 to 4 mg/kg, or most preferred from 0.001 to 2.5 mg/kg, determined as the mass of $Ca^{2+}$ ions in a sample of one kg of the softened seawater.

4. The softened seawater for use according to claim 2 or 3, wherein the period of time is in one of the following ranges; from 5 minutes to 200 hours, from 5 minutes to 100 hours, from 10 minutes to 48 hours, 20 minutes to 36 hours, more 30 minutes to 30 hours, from 60 minutes to 24 hours, or from 4 hours to 24 hours.

5. The softened seawater for use according to any preceding claim, wherein the treatment is made prophylactic by being intermittently repeated at regular intervals of from once a week to once a year, at regular intervals from once every two weeks to once every sixth month, at regular intervals once every month to once every fourth month, or at regular intervals once every second month to once every third month.

6. The softened seawater for use according to claim 1 or 2, wherein the fish to be treated is a salmonid, preferably Atlantic salmon (*Salmo salar*) and the method is aimed at reducing *Neoparamoeba perurans,* and wherein:

- the treatment comprises containing the salmonid in the softened seawater for a period of time chosen from one of the following ranges; from 1 hour to 48 hours, more preferably from 1 hour to 36 hours, more preferably from 2 hours to 24 hours and most preferably from 4 hours to 24 hours,
- the treatment is repeated at one of the following intervals; once each every sixth month, preferably every fourth month, more preferably every third month, more preferably every second month, more preferably every month, or most preferably every second week, and
- the softened seawater has:

- a salinity in the range from 1.0 g/kg to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
- a $Ca^{2+}$ content of $\leq$ 50 mg/kg, determined as the mass of $Ca^{2+}$ ions in a sample of one kg of the softened seawater.

7. The softened seawater for use according to claim 6, wherein the softened seawater has:

- a salinity in one of the following ranges: from 1 g/kg to 12.5 g/kg, from 1 g/kg to 10 g/kg, from 2 g/kg to 10 g/kg, or from 2 g/kg to 8 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
- a $Ca^{2+}$ content in one of the following ranges: from 0.001 to 30 mg/kg, from 0.001 to 15 mg/kg, from 0.1 to 10 mg/kg, from 0.1 to 8 mg/kg, from 0.1 to 6 mg/kg, from 0.1 to 4 mg/kg, or most preferred from 0.1 to 2.5 mg/kg, determined as the mass of $Ca^{2+}$ ions in a sample of one kg of the softened seawater.

8. The softened seawater for use according to claim 1 or 2, wherein the fish to be treated is a salmonid, preferably Atlantic salmon (*Salmo salar*) and the method is aimed at reducing *Lepeophtheirus salmonis* and/or a *Caligus* specie, and wherein:

- the treatment comprises containing the salmonid in the softened seawater for a period of time chosen from one of the following ranges; from 1 hour to 48 hours, more preferably from 1 hour to 36 hours, more preferably from 2 hours to 24 hours and most preferably from 4 hours to 24 hours,
- the treatment is repeated at one of the following intervals; once each every sixth month, preferably every fourth month, more preferably every third month, more preferably every second month, more preferably every month, or most preferably every second week, and
- the softened seawater has:

- a salinity in the range from 1.0 g/kg to 10 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
- a $Ca^{2+}$ content of $\leq$ 20 mg/kg, determined as the mass of $Ca^{2+}$ ions in a sample of one kg of the softened seawater.

9. The softened seawater for use according to claim 8, wherein the softened seawater has:

- a salinity in one of the following ranges: from 1 g/kg to 9 g/kg, from 1.5 g/kg to 8 g/kg, from 1.5 g/kg to 7 g/kg, or from 2 g/kg to 7 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
- a $Ca^{2+}$ content in one of the following ranges: from 0.1 to 20 mg/kg, from 0.1 to 15 mg/kg, from 0.2 to 10 mg/kg, from 0.2 to 6 mg/kg, from 0.2 to 5 mg/kg, from 0.2 to 4 mg/kg, or most preferred from 1 to 3 mg/kg, determined as the mass of $Ca^{2+}$ ions in a sample of one kg of the softened seawater.

10. A system, **characterised in that** the system comprises:

- a supply unit (2) of softened seawater having;

- a salinity in the range from 1.0 g/kg to 15 g/kg, determined as the total mass of $Na^+$, $K^+$, $Ca^{2+}$, $Mg^{2+}$, $Cl^-$, $HCO_3^-$, $CO_3^{2-}$, and $SO_4^{2-}$ ions being present in a sample of one kg of the softened seawater, and
- a $Ca^{2+}$ content of $\leq$ 100 mg/kg, determined as the mass of $Ca^{2+}$ ions in a sample of one kg of the softened seawater.

and
- a treatment zone (3) containing a volume of the softened seawater dimensioned to contain and sustain an intended number of marine fishes to be treated for a period of time,

and wherein the treatment zone is one of:

- a container at least partly filled with the softened seawater located either in-situ in a marine fish farm (14, 16) or external to the marine fish farm (14, 16),
- a top layer of a floating net-cage (14, 16) formed by a cage skirt/tarpaulin running along the upper peripheral part of the floating net-cage and where the top layer of water in the net-cage is softened seawater, or
- a barge or boat floating outside the net cages of marine fish farms having at least one compartment/container filled with softened seawater.

11. The system according to claim 10, wherein the $Ca^{2+}$- content of the softened seawater supplied by the supply unit (2) is in one of the following ranges; from 0.001 to 95 mg/kg, from 0.001 to 90 mg/kg, from 0.001 to 80 mg/kg, from 0.001 to 50 mg/kg, from 0.001 to 10 mg/kg, from 0.001 to 8 mg/kg, from 0.001 to 6 mg/kg, from 0.001 to 5 mg/kg, from 0.001 to 4 mg/kg, or most preferred from 0.001 to 2.5 mg/kg.

12. The system according to claim 10 or 11, wherein the salinity of the softened seawater supplied by the supply unit (2) is in one of the following ranges; from 1 g/kg to 12.5 g/kg, from 1 g/kg to 10 g/kg, from 2 g/kg to 15 g/kg, from 2 g/kg to 12.5 g/kg, or from 2 g/kg to 10 g/kg, or most preferably from 2 g/kg to 8 g/kg.

13. The system according to any of claims 10 to 12, wherein the supply unit (2) of the softened seawater comprises at least one nanofiltration membrane, preferably an anionic membrane having a pore size in the range of 1 - 10 nm, and where the softened seawater is produced by passing natural seawater through the at least one nanofiltration membrane

14. Softened seawater for use according to any of claims 1 - 9, wherein the softened seawater is administered in a system according to any of claims 10 - 13.

**Patentansprüche**

1. Enthärtetes Meerwasser zur Verwendung als Medikament,
   **dadurch gekennzeichnet, dass** das enthärtete Meerwasser aufweist:

   - einen Salzgehalt im Bereich von 1,0 bis 15 g/kg, bestimmt als die Gesamtmasse von $Na^+$-, $K^+$-, $Ca^{2+}$-, $Mg^{2+}$-, $Cl^-$-, $HCO_3^-$-, $CO_3^{2-}$- und $SO_4^{2-}$-Ionen, die in einer Probe von 1 kg des enthärteten Meerwassers vorhanden sind, und
   - einen $Ca^{2+}$-Gehalt von $\leq$ 100 mg/kg, bestimmt als die Masse von $Ca^{2+}$-Ionen in einer Probe von 1 kg des enthärteten Meerwassers,

   und dadurch, dass das enthärtete Meerwasser als Medikament zum Reduzieren von Calcium-sensitiven Ektoparasiten bei einem Meeresfisch, vorzugsweise bei einem oder mehreren von: *Neoparamoeba perurans* oder *Lepeophtheirus salmonis,* verwendet wird.

2. Enthärtetes Meerwasser zur Verwendung in einem Verfahren zur Behandlung von Meeresfischen gegen Calcium-sensitive Ektoparasiten durch Halten des Fisches für einen Zeitraum in dem enthärteten Meerwasser, wobei der zu behandelnde Fisch ein Lachsfisch, vorzugsweise Atlantischer Lachs (*Salmo salar*) ist und das Verfahren auf ein Reduzieren von *Neoparamoeba perurans* oder *Lepeophtheirus salmonis* abzielt, und wobei das enthärtete Meerwasser aufweist:

   - einen Salzgehalt im Bereich von 1,0 bis 15 g/kg, bestimmt als die Gesamtmasse von $Na^+$-, $K^+$-, $Ca^{2+}$-, $Mg^{2+}$-,

Cl⁻-, HCO₃⁻-, CO₃²⁻- und SO₄²⁻-Ionen, die in einer Probe von 1 kg des enthärteten Meerwassers vorhanden sind, und
- einen $Ca^{2+}$-Gehalt von $\leq$ 100 mg/kg, bestimmt als die Masse von $Ca^{2+}$-Ionen in einer Probe von 1 kg des enthärteten Meerwassers.

**3.** Enthärtetes Meerwasser zur Verwendung nach einem der Ansprüche 1 bis 2, wobei:

- der Salzgehalt des enthärteten Meerwassers in einem der folgenden Bereiche liegt: von 1 g/kg bis 12,5 g/kg, von 1 g/kg bis 10 g/kg, von 2 g/kg bis 15 g/kg, von 2 g/kg bis 12,5 g/kg oder von 2 g/kg bis 10 g/kg oder besonders bevorzugt von 2 g/kg bis 8 g/kg, bestimmt als die Gesamtmasse von $Na^+$-, $K^+$-, $Ca^{2+}$-, $Mg^{2+}$-, $Cl^-$-, $HCO_3^-$-, $CO_3^{2-}$- und $SO_4^{2-}$-Ionen, die in einer Probe von 1 kg des enthärteten Meerwassers vorhanden sind, und
- der $Ca^{2+}$-Gehalt des enthärteten Meerwassers in einem der folgenden Bereiche liegt: von 0,001 bis 95 mg/kg, von 0,001 bis 90 mg/kg, von 0,001 bis 80 mg/kg, von 0,001 bis 50 mg/kg, von 0,001 bis 10 mg/kg, von 0,001 bis 8 mg/kg, von 0,001 bis 6 mg/kg, von 0,001 bis 5 mg/kg, von 0,001 bis 4 mg/kg oder besonders bevorzugt von 0,001 bis 2,5 mg/kg, bestimmt als die Masse von $Ca^{2+}$-Ionen in einer Probe von 1 kg des enthärteten Meerwassers.

**4.** Enthärtetes Meerwasser zur Verwendung nach Anspruch 2 oder 3, wobei der Zeitraum in einem der folgenden Bereiche liegt: von 5 Minuten bis 200 Stunden, von 5 Minuten bis 100 Stunden, von 10 Minuten bis 48 Stunden, 20 Minuten bis 36 Stunden, mehr als 30 Minuten bis 30 Stunden, von 60 Minuten bis 24 Stunden oder von 4 Stunden bis 24 Stunden.

**5.** Enthärtetes Meerwasser zur Verwendung nach einem vorangehenden Anspruch, wobei die Behandlung prophylaktisch durchgeführt wird, indem sie in regelmäßigen Intervallen von einmal pro Woche bis einmal pro Jahr, in regelmäßigen Intervallen von einmal alle zwei Wochen bis einmal jeden sechsten Monat, in regelmäßigen Intervallen von einmal pro Monat bis einmal jeden vierten Monat oder in regelmäßigen Intervallen von einmal jeden zweiten Monat bis einmal jeden dritten Monat intermittierend wiederholt wird.

**6.** Enthärtetes Meerwasser zur Verwendung nach Anspruch 1 oder 2, wobei der zu behandelnde Fisch ein Lachsfisch, vorzugsweise Atlantischer Lachs (*Salmo salar*) ist und das Verfahren auf ein Reduzieren von *Neoparamoeba perurans* abzielt und wobei:

- die Behandlung Halten des Lachsfischs in dem enthärteten Meerwasser für einen Zeitraum, ausgewählt aus einem der folgenden Bereiche: von 1 Stunde bis 48 Stunden, bevorzugter von 1 Stunde bis 36 Stunden, bevorzugter von 2 Stunden bis 24 Stunden und am bevorzugtesten von 4 Stunden bis 24 Stunden, umfasst,

- die Behandlung in einem der folgenden Intervalle wiederholt wird: einmal jeden sechsten Monat, vorzugsweise jeden vierten Monat, bevorzugter jeden dritten Monat, bevorzugter jeden zweiten Monat, bevorzugter jeden Monat oder am bevorzugtesten jede zweite Woche, und
- das enthärtete Meerwasser aufweist:

  ∘ einen Salzgehalt im Bereich von 1,0 bis 15 g/kg, bestimmt als die Gesamtmasse von $Na^+$-, $K^+$-, $Ca^{2+}$-, $Mg^{2+}$-, $Cl^-$-, $HCO_3^-$-, $CO_3^{2-}$- und $SO_4^{2-}$-Ionen, die in einer Probe von 1 kg des enthärteten Meerwassers vorhanden sind, und
  ∘ einen $Ca^{2+}$-Gehalt von $\leq$ 50 mg/kg, bestimmt als die Masse von $Ca^{2+}$-Ionen in einer Probe von 1 kg des enthärteten Meerwassers.

**7.** Enthärtetes Meerwasser zur Verwendung nach Anspruch 6, wobei das enthärtete Meerwasser aufweist:

- einen Salzgehalt in einem der folgenden Bereiche: von 1 g/kg bis 12,5 g/kg, von 1 g/kg bis 10 g/kg, von 2 g/kg bis 10 g/kg oder von 2 g/kg bis 8 g/kg, bestimmt als die Gesamtmasse von $Na^+$-, $K^+$-, $Ca^{2+}$-, $Mg^{2+}$-, $Cl^-$-, $HCO_3^-$-, $CO_3^{2-}$- und $SO_4^{2-}$-Ionen, die in einer Probe von 1 kg des enthärteten Meerwassers vorhanden sind, und
- einen $Ca^{2+}$-Gehalt in einem der folgenden Bereiche: von 0,001 bis 30 mg/kg, von 0,001 bis 15 mg/kg, von 0,1 bis 10 mg/kg, von 0,1 bis 8 mg/kg, von 0,1 bis 6 mg/kg, von 0,1 bis 4 mg/kg oder am bevorzugtesten von 0,1 bis 2,5 mg/kg, bestimmt als die Masse von $Ca^{2+}$-Ionen in einer Probe von 1 kg des enthärteten Meerwassers.

**8.** Enthärtetes Meerwasser zur Verwendung nach Anspruch 1 oder 2, wobei der zu behandelnde Fisch ein Lachsfisch, vorzugsweise Atlantischer Lachs (*Salmo salar*) ist und das Verfahren auf ein Reduzieren von *Lepeophtheirus sal-*

*monis* und/oder einer Caligus-Spezies abzielt,
und wobei:

- die Behandlung Halten des Lachsfisches in dem enthärteten Meerwasser für einen Zeitraum, ausgewählt aus einem der folgenden Bereiche: von 1 Stunde bis 48 Stunden, bevorzugter von 1 Stunde bis 36 Stunden, bevorzugter von 2 Stunden bis 24 Stunden und am bevorzugtesten von 4 Stunden bis 24 Stunden, umfasst,
- die Behandlung in einem der folgenden Intervalle wiederholt wird: einmal jeden sechsten Monat, vorzugsweise jeden vierten Monat, bevorzugter jeden dritten Monat, bevorzugter jeden zweiten Monat, bevorzugter jeden Monat oder am bevorzugtesten jede zweite Woche, und
- das enthärtete Meerwasser aufweist:

  - einen Salzgehalt im Bereich von 1,0 bis 10 g/kg, bestimmt als die Gesamtmasse von $Na^+$-, $K^+$-, $Ca^{2+}$-, $Mg^{2+}$-, $Cl^-$-, $HCO_3^-$, $CO_3^{2-}$-und $SO_4^{2-}$-Ionen, die in einer Probe von 1 kg des enthärteten Meerwassers vorhanden sind, und
  - einen $Ca^{2+}$-Gehalt von $\leq$ 20 mg/kg, bestimmt als die Masse von $Ca^{2+}$-Ionen in einer Probe von 1 kg des enthärteten Meerwassers.

9. Enthärtetes Meerwasser zur Verwendung nach Anspruch 8, wobei das enthärtete Meerwasser aufweist:

   - einen Salzgehalt in einem der folgenden Bereiche: von 1 g/kg bis 9 g/kg, von 1,5 g/kg bis 8 g/kg, von 1,5 g/kg bis 7 g/kg oder von 2 g/kg bis 7 g/kg, bestimmt als die Gesamtmasse von $Na^+$-, $K^+$-, $Ca^{2+}$-, $Mg^{2+}$-, $Cl^-$-, $HCO_3^-$-, $CO_3^{2-}$- und $SO_4^{2-}$-Ionen, die in einer Probe von 1 kg des enthärteten Meerwassers vorhanden sind, und
   - einen $Ca^{2+}$-Gehalt in einem der folgenden Bereiche: von 0,1 bis 20 mg/kg, von 0,1 bis 15 mg/kg, von 0,2 bis 10 mg/kg, von 0,2 bis 6 mg/kg, von 0,2 bis 5 mg/kg, von 0,2 bis 4 mg/kg oder am bevorzugtesten von 1 bis 3 mg/kg, bestimmt als die Masse von $Ca^{2+}$-Ionen in einer Probe von 1 kg des enthärteten Meerwassers.

10. System, **dadurch gekennzeichnet, dass** das System umfasst:

    - eine Zuführeinheit (2) für enthärtetes Meerwasser, das aufweist:

      - einen Salzgehalt im Bereich von 1,0 bis 15 g/kg, bestimmt als die Gesamtmasse von $Na^+$-, $K^+$-, $Ca^{2+}$-, $Mg^{2+}$-, $Cl^-$-, $HCO_3^-$, $CO_3^{2-}$- und $SO_4^{2-}$-Ionen, die in einer Probe von 1 kg des enthärteten Meerwassers vorhanden sind, und
      - einen $Ca^{2+}$-Gehalt von $\leq$ 100 mg/kg, bestimmt als die Masse von $Ca^{2+}$-Ionen in einer Probe von 1 kg des enthärteten Meerwassers,

    und

      - eine Behandlungszone (3), die ein Volumen des enthärteten Meerwassers enthält, das dimensioniert ist, um die vorgesehene Anzahl von Meeresfischen, die für einen Zeitraum behandelt werden soll, aufzunehmen und zu erhalten,

    und wobei die Behandlungszone eins der folgenden ist:

      - ein Behälter, der wenigstens teilweise mit dem enthärteten Meerwasser gefüllt ist, der sich entweder vor Ort in einer Meeresfischfarm (14, 16) oder außerhalb der Meeresfischfarm (14, 16) befindet,
      - eine obere Schicht eines schwimmenden Netzkäfigs (14, 16), die durch einen Käfigrand/eine Plane gebildet wird, der/die entlang des oberen peripheren Teils des schwimmenden Netzkäfigs verläuft, und wobei die obere Wasserschicht im Netzkäfig enthärtetes Meerwasser ist, oder
      - ein Lastkahn oder Boot, der/das außerhalb der Netzkäfige von Meeresfischfarmen schwimmt und mindestens eine Kammer/einen Behälter, gefüllt mit enthärtetem Meerwasser, aufweist.

11. System nach Anspruch 10, wobei der $Ca^{2+}$-Gehalt des durch die Zuführeinheit (2) zugeführten enthärteten Meerwassers in einem der folgenden Bereiche liegt: von 0,001 bis 95 mg/kg, von 0,001 bis 90 mg/kg, von 0,001 bis 80 mg/kg, von 0,001 bis 50 mg/kg, von 0,001 bis 10 mg/kg, von 0,001 bis 8 mg/kg, von 0,001 bis 6 mg/kg, von 0,001 bis 5 mg/kg, von 0,001 bis 4 mg/kg oder besonders bevorzugt von 0,001 bis 2,5 mg/kg.

12. System nach Anspruch 10 oder 11, wobei der Salzgehalt des durch die Zuführeinheit (2) zugeführten enthärteten Meerwassers in einem der folgenden Bereiche liegt:
von 1 g/kg bis 12,5 g/kg, von 1 g/kg bis 10 g/kg oder von 2 g/kg bis 15 g/kg, von 2 g/kg bis 12,5 g/kg oder von 2g/kg bis 10 g/kg oder am bevorzugtesten von 2 g/kg bis 8 g/kg.

**13.** System nach einem der Ansprüche 10 bis 12, wobei die Zuführeinheit (2) für das enthärtete Meerwasser wenigstens eine Nanofiltrationsmembran, vorzugsweise eine anionische Membran mit einer Porengröße im Bereich von 1 - 10 nm umfasst und wobei das enthärtete Meerwasser hergestellt wird, indem natürliches Meerwasser durch die wenigstens eine Nanofiltrationsmembran geleitet wird.

**14.** Enthärtetes Meerwasser zur Verwendung nach einem der Ansprüche 1 - 9, wobei das enthärtete Meerwasser in einem System nach einem der Ansprüche 10 - 13 verabreicht wird.

**Revendications**

1. Eau de mer adoucie pour utilisation comme médicament,
   **caractérisée en ce que** l'eau de mer adoucie a :

   - une salinité dans la plage allant de 1,0 à 15 g/kg, déterminée comme la masse totale d'ions $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
   - une teneur en $Ca^{2+} \leq 100$ mg/kg, déterminée comme la masse d'ions $Ca^{2+}$ dans un échantillon d'un kg de l'eau de mer adoucie,

   et **en ce que** l'eau de mer adoucie est utilisée comme médicament pour réduire les ectoparasites sensibles au calcium sur un poisson marin, de préférence un ou plusieurs parmi : *Neoparamoeba perurans* ou *Lepeophtheirus salmonis.*

2. Eau de mer adoucie pour utilisation dans une méthode de traitement de poissons marins contre les ectoparasites sensibles au calcium en contenant les poissons pendant une période de temps dans l'eau de mer adoucie, dans laquelle le poisson à traiter est un salmonidé, de préférence le saumon de l'Atlantique (*Salmo solar*) et la méthode vise à réduire *Neoparamoeba perurans* ou *Lepeophtheirus salmonis,* et dans laquelle l'eau de mer adoucie a :

   - une salinité dans la plage allant de 1,0 à 15 g/kg, déterminée comme la masse totale d'ions $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
   - une teneur en $Ca^{2+} \leq 100$ mg/kg, déterminée comme la masse d'ions $Ca^{2+}$ dans un échantillon d'un kg de l'eau de mer adoucie.

3. Eau de mer adoucie pour utilisation selon l'une quelconque des revendications 1 à 2, dans laquelle :

   - la salinité de l'eau de mer adoucie est dans l'une des plages suivantes : de 1 g/kg à 12,5 g/kg, de 1 g/kg à 10 g/kg, de 2 g/kg à 15 g/kg, de 2 g/kg à 12,5 g/kg ou de 2 g/kg à 10 g/kg, ou de manière préférée entre toutes de 2 g/kg à 8 g/kg, déterminée comme la masse totale des ions $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
   - la teneur en $Ca^{2+}$ de l'eau de mer adoucie est dans l'une des plages suivantes : de 0,001 à 95 mg/kg, de 0,001 à 90 mg/kg, de 0,001 à 80 mg/kg, de 0,001 à 50 mg/kg, de 0,001 à 10 mg/kg, de 0,001 à 8 mg/kg, de 0,001 à 6 mg/kg, de 0,001 à 5 mg/kg, de 0,001 à 4 mg/kg, ou de manière préférée entre toutes de 0,001 à 2,5 mg/kg, déterminée comme la masse d'ions $Ca^{2+}$ dans un échantillon d'un kg de l'eau de mer adoucie.

4. Eau de mer adoucie pour utilisation selon la revendication 2 ou 3, dans laquelle la période de temps est dans l'une des plages suivantes : de 5 minutes à 200 heures, de 5 minutes à 100 heures, de 10 minutes à 48 heures, de 20 minutes à 36 heures, plus de 30 minutes à 30 heures, de 60 minutes à 24 heures ou de 4 heures à 24 heures.

5. Eau de mer adoucie pour utilisation selon l'une quelconque des revendications précédentes, dans laquelle le traitement est rendu prophylactique en étant répété par intermittence à intervalles réguliers d'une fois par semaine à une fois par an, à intervalles réguliers d'une fois toutes les deux semaines à une fois tous les six mois, à intervalles réguliers d'une fois tous les mois à une fois tous les quatre mois ou à intervalles réguliers d'une fois tous les deux mois à une fois tous les trois mois.

**6.** Eau de mer adoucie pour utilisation selon la revendication 1 ou 2, dans laquelle le poisson à traiter est un salmonidé, de préférence le saumon de l'Atlantique (*Salmo salar*) et le traitement vise à réduire *Neoparamoeba perurans,* et dans laquelle :

- le traitement consiste à contenir le salmonidé dans de l'eau de mer adoucie pendant une période de temps choisie parmi l'une des plages suivantes : de 1 heure à 48 heures, plus préférablement de 1 heure à 36 heures, plus préférablement de 2 heures à 24 heures et de manière préférée entre toutes de 4 heures à 24 heures,
- le traitement est répété à l'un des intervalles suivants; une fois tous les six mois, de préférence tous les quatre mois, plus préférablement tous les trois mois, plus préférablement tous les deux mois, plus préférablement tous les mois ou de manière préférée entre toutes toutes les deux semaines, et
- l'eau de mer adoucie a :

- une salinité dans la plage allant de 1,0 à 15 g/kg, déterminée comme la masse totale d'ions $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
- une teneur en $Ca^{2+} \leq 50$ mg/kg, déterminée comme la masse d'ions $Ca^{2+}$ dans un échantillon d'un kg de l'eau de mer adoucie.

**7.** Eau de mer adoucie pour utilisation selon la revendication 6, dans laquelle l'eau de mer adoucie a :

- une salinité dans l'une des plages suivantes : de 1 g/kg à 12,5 g/kg, de 1 g/kg à 10 g/kg, de 2 g/kg à 10 g/kg ou de 2 g/kg à 8 g/kg, déterminée comme la masse totale d'ions $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
- une teneur en $Ca^{2+}$ dans l'une des plages suivantes : de 0,001 à 30 mg/kg, de 0,001 à 15 mg/kg, de 0,1 à 10 mg/kg, de 0,1 à 8 mg/kg, de 0,1 à 6 mg/kg, de 0,1 à 4 mg/kg ou de manière préférée entre toutes de 0,1 à 2,5 mg/kg, déterminée comme la masse d'ions $Ca^{2+}$ dans un échantillon d'un kg de l'eau de mer adoucie.

**8.** Eau de mer adoucie pour utilisation selon revendication 1 ou 2, dans laquelle le poisson à traiter est un salmonidé, de préférence le saumon de l'Atlantique (*Salmo salar*) et la méthode vise à réduire *Lepeophtheirus salmonis* et/ou une espèce *Caligus,* et dans laquelle :

- le traitement consiste à contenir le salmonidé dans l'eau de mer adoucie pendant une période de temps choisie parmi l'une des plages suivantes : de 1 heure à 48 heures, plus préférablement de 1 heure à 36 heures, plus préférablement de 2 heures à 24 heures et de manière préférée entre toutes de 4 heures à 24 heures,
- le traitement est répété à l'un des intervalles suivants : une fois tous les six mois, de préférence tous les quatre mois, plus préférablement tous les trois mois, plus préférablement tous les deux mois, plus préférablement tous les mois ou manière préférée entre toutes toutes les deux semaines, et
- l'eau de mer adoucie a :

- une salinité dans la plage allant de 1,0 à 10 g/kg, déterminée comme la masse totale d'ions $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
- une teneur en $Ca^{2+} \leq 20$ mg/kg, déterminée comme la masse d'ions $Ca^{2+}$ dans un échantillon d'un kg de l'eau de mer adoucie.

**9.** Eau de mer adoucie pour utilisation selon la revendication 8, dans laquelle l'eau de mer adoucie a :

- une salinité dans l'une des plages suivantes : de 1 g/kg à 9 g/kg, de 1,5 g/kg à 8 g/kg, de 1,5 g/kg à 7 g/kg ou de 2 g/kg à 7 g/kg, déterminée comme la masse totale de $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
- une teneur en $Ca^{2+}$ dans l'une des plages suivantes : de 0,1 à 20 mg/kg, de 0,1 à 15 mg/kg, de 0,2 à 10 mg/kg, de 0,2 à 6 mg/kg, de 0,2 à 5 mg/kg, de 0,2 à 4 mg/kg ou de manière préférée entre toutes de 1 à 3 mg/kg, déterminée comme la masse d'ions $Ca^{2+}$ dans un échantillon d'un kg de l'eau de mer adoucie.

**10.** Système, **caractérisé en ce que** le système comprend :

- une unité d'alimentation (2) en eau de mer adoucie ayant :

- une salinité dans la plage allant de 1,0 à 15 g/kg, déterminée comme la masse totale d'ions $Na^+$, $K^+$, $Ca^{2+}$, $Mg^{2+}$, $Cl^-$, $HCO_3^-$, $CO_3^{2-}$ et $SO_4^{2-}$ présents dans un échantillon d'un kg de l'eau de mer adoucie, et
- une teneur en $Ca^{2+} \leq 100$ mg/kg, déterminée comme la masse d'ions $Ca^{2+}$ dans un échantillon d'un kg de l'eau de mer adoucie,

et
- une zone de traitement (3) contenant un volume de l'eau de mer adoucie dimensionné pour contenir et maintenir un nombre prévu de poissons marins à traiter pendant une période de temps,

et dans lequel la zone de traitement est l'une parmi :

- un contenant au moins partiellement rempli avec de l'eau de mer adoucie située soit in situ, soit dans une ferme piscicole marine (14, 16) ou à l'extérieure de la ferme piscicole marine (14, 16),
- une couche supérieure d'un enclos à filet flottant (14, 16) formée par une jupe cage/bâche s'étendant le long de la partie périphérique supérieure de l'enclos à filet flottant et où la couche supérieure de l'eau dans l'enclos à filet flottant est de l'eau de mer adoucie, ou
- une péniche ou un bateau flottant à l'extérieur des enclos à filet des fermes piscicoles marines ayant au moins un compartiment/récipient rempli avec de l'eau de mer adoucie.

11. Système selon la revendication 10, dans lequel la teneur en $Ca^{2+}$ de l'eau de mer adoucie fournie par l'unité d'alimentation (2) est dans l'une des plages suivantes : de 0,001 à 95 mg/kg, de 0,001 à 90 mg/kg, de 0,001 à 80 mg/kg, de 0,001 à 50 mg/kg, de 0,001 à 10 mg/kg, de 0,001 à 8 mg/kg, de 0,001 à 6 mg/kg, de 0,001 à 5 mg/kg, de 0,001 à 4 mg/kg ou de manière préférée entre toutes de 0,001 à 2,5 mg/kg.

12. Système selon la revendication 10 ou 11, dans lequel la salinité de l'eau de mer adoucie fournie par l'unité d'alimentation (2) est dans l'une des plages suivantes : de 1 g/kg à 12,5 g/kg, de 1 g/kg à 10 g/kg, de 2 g/kg à 15 g/kg, de 2 g/kg à 12,5 g/kg ou de 2 g/kg à 10 g/kg ou de manière préférée entre toutes de 2 g/kg à 8 g/kg.

13. Système selon l'une quelconque des revendications 10 à 12, dans lequel l'unité d'alimentation (2) en eau de mer adoucie comprend au moins une membrane de nanofiltration, de préférence une membrane anionique ayant une taille de pores dans la plage de 1 à 10 nm, et où l'eau de mer adoucie est produite en faisant passer de l'eau de mer naturelle à travers la au moins une membrane de nanofiltration.

14. Eau de mer adoucie pour utilisation selon l'une quelconque des revendications 1 à 9, dans laquelle l'eau de mer adoucie est administrée dans un système selon l'une quelconque des revendications 10 à 13.

Figure 1

Figure 2

Figure 3

Figure 4

a)

b)

Figure 5

Figure 6

Figure 7

Figure 8

Figure 9

EP 3 629 738 B1

Figure 10

a)

**7h bathing test 1: juveniles and pre-adults**

b)

**7h bathing test 2: mature adults**

Figure 11

EP 3 629 738 B1

Figure 12

EP 3 629 738 B1

a)

b)

Severity of AGD (score * No. affected surfaces) (+ SD)

Average gill score per surface (+ SD)

Pre | Post-bath | 24h post-bath

Pre    SW    FW    Sol A    Sol C

Figure 13

Figure 14

Figure 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008069261 A
- NO 334487
- NO 333846
- US 20120152721 A

### Non-patent literature cited in the description

- **POWELL ; KRISTENSEN.** Freshwater treatment of amoebic gill disease and sea-lice in seawater salmon production; considerations of water chemistry and fish welfare. *NIVA,* 2014
- **LIMA et al.** Involvement of contractile vacuoles in the osmoregulation process of the marine parasitic amoeba Neoparamoeba perurans. *Journal of Fish Diseases,* 2015
- Chemical Principles. W.B. Saunders Company, 1977, 299
- **TAYLOR RS ; MULLER WJ ; COOK MT ; KUBE PD ; ELLIOTT NG.** Gill observations in Atlantic salmon (Salmo salar, L.) during repeated amoebic gill disease (AGD) field exposure and survival challenge. *Aquaculture,* 2009, vol. 290 (1-2), 1-8
- Exploitation of Water Chemistry in the Treatment of Amoebic Gill Desease. **TIMOTHY J. GREEN.** Master thesis, School of Agriculture. University of Tasmania, October 2003
- **MARK D. POWELL et al.** In vitro survival and the effect of water chemistry and oxidative chemical treatments on isolated gill amoeba from AGD-affected Atlantic salmon. *AQUACULTURE,* 2003, 135-144
- **HARRIET PERRY et al.** Calcium concentration in seawater and exoskeletal calcification in the blue crab, Callinectes sapidus. *Aquaculture,* 2001, vol. 198 (3-4), 197-208
- **YUEFEI SONG et al.** Performance of UF NF integrated membrane process for seawater softening. *DESALINATION,* 2011, vol. 276 (1), 109-116